(19) Europäisches Patentamt
European Patent Office
Office européen des brevets

(11) EP 4 574 268 A1

# (12) EUROPEAN PATENT APPLICATION

(43) Date of publication:
**25.06.2025 Bulletin 2025/26**

(21) Application number: **23315459.0**

(22) Date of filing: **18.12.2023**

(51) International Patent Classification (IPC):
***B04B 5/04*** (2006.01) ***B04B 5/02*** (2006.01)
***B04B 7/08*** (2006.01) ***B04B 11/02*** (2006.01)
***B04B 15/06*** (2006.01) ***B01L 3/00*** (2006.01)
***C12M 1/26*** (2006.01)

(52) Cooperative Patent Classification (CPC):
**B04B 5/02; B01L 3/5021; B01L 13/02; B04B 5/0442; B04B 7/08; B04B 11/02; B04B 15/06; C12M 33/10**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **Thermo Electron SAS**
**91140 Villebon-sur-Yvette (FR)**
- **Thermo Electron LED GmbH**
**37520 Osterode (DE)**

(72) Inventors:
- **Limon, Benoit**
**91140 Villebon-sur-Yvette (FR)**
- **Langer, Daniel**
**37520 Osterode (DE)**

(74) Representative: **Boult Wade Tennant LLP**
**Salisbury Square House**
**8 Salisbury Square**
**London EC4Y 8AP (GB)**

## (54) CENTRIFUGE CONTAINER FOR CLARIFYING A LIQUID AND METHOD THEREOF

(57) A centrifuge container for clarifying a liquid containing suspended particles is provided. The centrifuge container comprises: a connection end for connection to a centrifuge rotor; and an outer end for concentration of particles. The outer end is spaced from the connection end in a first direction along a longitudinal axis of the centrifuge container. The centrifuge container further comprises: a first fluid port at the connection end for flowing a liquid containing suspended particles into the centrifuge container; a second fluid port at the connection end for flowing the liquid containing suspended particles from the centrifuge container; and a flushing tube extending from the connection end towards the outer end, the flushing tube for delivering a flushing fluid to flush concentrated particles from the outer end.

Fig. 1

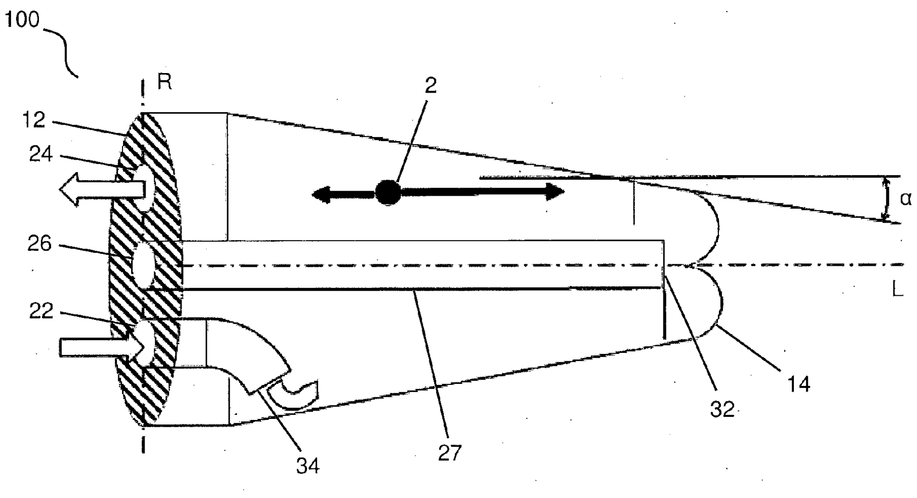


EP 4 574 268 A1

## Description

### Background

The present specification relates to methods of clarifying a liquid containing suspended particles, a centrifuge container, and associated aspects.

Clarification of particle-containing liquids if a common process, for examples in bioprocessing. To do so, depth-filtration or pelleting-bowl centrifugation are the current methods to remove small particles (<50 μm, especially <10 μm, or < 1μm, or even 0.1 μm or less) from a suspension. These methods require different filters with different pore sizes according to the particle size that needs to be removed.

Furthermore, the method of depth-filtration is cost-intensive as the filters needs to be renewed depending on material and special liquid for flushing is required to maintain operation. Both methods only allow for the use of the filtered material by a complicated process in cleaning the clogged filters. The filter portion must typically be opened, which often requires manual input. This is particularly problematic for bioprocessing since exposure between then manual input and the system needs to be prevented.

Pelleting drum rotors are used in certain continuous-flow clarification systems. However, these can only be used in an open system and hence result in a risk of contamination. There is also no simple recovery of the pelletised material.

Further examples of centrifuge systems include the Beckman JCF-Z. This requires the rotor to be disassembled and scraped in order to remove any build-up material. This is complex and results in the system being non-operational for a period of time.

There are also disc-stack type centrifuges such as the Huading Separator. Again, this cannot be operated in a closed system and so introduces risks of contamination. There is also no pelletisation of material for recovery.

There is therefore a need for an improved method of clarifying a liquid, and a centrifuge container.

### Summary

A method of clarifying a liquid containing suspended particles is provided. The method comprises the steps of: (a) flowing a liquid containing suspended particles into a centrifuge container arranged on a centrifuge rotor; (b) rotating the rotor at a first rotational speed to concentrate the suspended particles at an outer end of the centrifuge container as a concentrated pellet; (c) rotating the rotor at a second rotational speed and removing the liquid from the centrifuge container; and (d) flushing the concentrated pellet from the centrifuge container.

This method effectively produces both a clarified liquid (supernatant) as well as a pellet of the suspended particles. Either or both can then be taken for further processing.

The first rotational speed and the second rotational speed may be in different directions to one another. This can help mitigate negative effects of Coriolis forces, or indeed take advantage of these forces to improve the collection and/or recovery steps.

The second rotational speed may be slower than the first rotational speed. As noted above, they may be in diffident directions and so this is the modulus or absolute value of each speed. In the concentration step the g-forces need to be high in order to sediment the suspended particles. Then the removal of the liquid and/or the concentrated pellet then takes place with a lower g-force.

The first rotational speed may be a maximum operating speed for the centrifuge rotor. This is either a highest speed for the particular method and/or a maximum safe operating speed for the apparatus used. High speed rotation is needed to separate out smaller particles.

The method may further comprise the step of: recovering the flushed pellet. For example, the pellet may be collected in a container or the like. The recovered pellet may then be used in further processing.

The liquid may be flowed into the centrifuge container via a first fluid port in step (a) and the liquid may be removed from the centrifuge container via the first fluid port in step (c). This is an effective arrangement to achieve the method.

The concentrated pellet may be flushed from the centrifuge container via the first fluid port. This is an effective arrangement to achieve the method.

The method may further comprise between steps (b) and (c) the steps of: (b-i) flowing the liquid from the centrifuge container; and (b-ii) flowing the liquid back into the centrifuge container, while rotating the rotor at a third rotational speed. This repetition ensures that all suspended particles may be pelletised.

The liquid may be flowed from the centrifuge container via a second fluid port in step (b-i) and the liquid may be flowed back into the centrifuge container via the first fluid port in step (b-ii). This is an effective arrangement to achieve the recirculation.

The third rotational speed may be the same as the first rotational speed. This is a highspeed which effectively pelletises small particles.

The method may further comprise the steps of: detecting an amount of suspended particles in the liquid flowing from the centrifuge container and/or the liquid flowing into the centrifuge container; repeating steps (b-i) and (b-ii) until the amount of suspended particles is below a threshold, and then proceeding to step (c). This allows for all particles (within a tolerance) to be removed from the liquid.

The method may further comprise the step of: flowing a gas into the centrifuge container during step (c) and/or (d). The gas flowing in can help force liquid from the centrifuge container. This flowing of the gas can be by directly pumping the gas into the centrifuge container, or the gas can be drawn into the centrifuge container by removing material from the centrifuge container and thereby generating a negative pressure therein.

The gas may be flowed into the centrifuge container via the second fluid port. This is an effective arrangement to achieve the method, while avoiding gas bubbles.

The method may further comprise the steps of: estimating a settling time based on one or more of: physical properties of the suspended particles; dimensions of the centrifuge container; physical properties of the liquid; the first rotational speed, wherein step (b) is performed for the estimated settling time before step (c). This helps ensure that all suspended particles are pelletised.

The method may further comprise the steps of: removing the centrifuge container from the centrifuge rotor; attaching a new centrifuge container to the centrifuge rotor; and repeating steps (a) to (d). This is a single-use method whereby a new centrifuge container is used for each operation.

A centrifuge container for clarifying a liquid containing suspended particles is provided. The centrifuge container comprises: a connection end for connection to a centrifuge rotor; an outer end for concentration of particles, the outer end spaced from the connection end in a first direction along a longitudinal axis of the centrifuge container; a first fluid port at the connection end for flowing a liquid containing suspended particles into the centrifuge container; a second fluid port at the connection end for flowing the liquid containing suspended particles from the centrifuge container; and a flushing tube extending from the connection end towards the outer end, the flushing tube for delivering a flushing fluid to flush concentrated particles from the outer end.

This centrifuge container is particularly effective for clarifying liquids containing suspended particles, particularly to allow for efficient recovery of either or both of the liquid and the particles.

The centrifuge container may taper in the first direction. This tapering helps to prevent flow separation.

The taper angle may be 30 degrees or less, preferably 16 degrees or less. For example, a total taper angle may be approximately 8 degrees. These can be effective angles for preventing flow separation which allows for an even flow distribution. If flow separation from the container walls does occur, there is an increased risk of eddies and dead zones. These then collect particles which hampers the concentration.

The taper angle may be at least 3 degrees. These can be effective angles for preventing flow separation.

The taper angle may be such that fluid flow from the outer end to the connection end does not have flow separation.

The centrifuge container may comprise a cylindrical section at the connection end, followed by a tapering section which tapers in the first direction. Such a shape provides an effective centrifuge container.

The cylindrical section may have a shorter length in the first direction than the tapering section. The tapering section therefore has the majority of the fluid flow in the centrifuge container.

The tapering section may be at least twice as long as the cylindrical section in the first direction, preferably at least four times as long. The tapering section therefore has the majority of the fluid flow in the centrifuge container.

The outer end of the centrifuge container may be at least partially shaped as a torus. A torus is a surface of revolution generated by revolving a circle in three-dimensional space one full revolution about a torus axis that is coplanar with the circle. The torus or toroidal shape helps to redirect fluid flow while maintaining momentum and having minimal pressure loss.

The outer end of the centrifuge container may be a semi-torus. A semi-torus is formed by splitting a torus in half along a plane which is perpendicular to the torus axis. In other words, the plane passes through a centre of the circle used to define the torus about the circle's entire revolution about the torus axis. This effectively leaves a semi-circular shape which is beneficial for redirecting the fluid flow.

The torus may be a horn torus. A horn torus is a torus where the torus axis of revolution is tangent to the circle used to define the torus. This is an effective torus shape for redirecting fluid flow in this manner.

A centre of the torus may be aligned with an outlet of the flushing tube. This helps to redirect flushing fluid flow efficiently.

The outlet of the flushing tube may be adjacent to the centre of the torus, preferably the outlet of the flushing tube is co-planar with the centre of the torus. This helps to redirect flushing fluid flow efficiently.

The torus may have a minor diameter of at least 0.5 times a diameter of the flushing tube, preferably at least 1 times, more preferably at least 1.5 times. This helps to redirect flushing fluid flow efficiently.

The torus may have a major diameter of at least 1 times a diameter of the flushing tube, preferably at least 1.5 times, more preferably at least 2 times. This helps to redirect flushing fluid flow efficiently.

The first fluid port, the second fluid port and the flushing tube may be spaced from one another in a second direction, transverse to the first direction. That is, they may be arranged in a line. This is an efficient centrifuge container arrangement.

The flushing tube may be between the first fluid port and the second fluid port in the second direction. The flushing tube is between the two ports, to reflect the respective use of each port.

The second fluid port may comprise a declined section extending towards an inner wall or surface of the centrifuge container. In other words, the declined section extends transverse to the first direction and the second direction. The declined section extends, in use, downwardly towards an inner wall/surface of the centrifuge container. This means that in use material which tends to pool at lower surface under gravity can be removed using the declined section.

The flushing tube may be aligned with the longitudinal axis of the centrifuge container. The pellet may form around the longitudinal axis of the centrifuge container and so directing the flushing flow here can help to effectively remove the pellet.

An inner surface and/or an outer surface of the centrifuge container may be rotationally symmetric about the longitudinal axis of the centrifuge container. This excludes, for example, the declined portion and/or the flushing tube. It is the overall shape of the inner surface or outer surface of the centrifuge container. A rotationally symmetric centrifuge container is easier to manufacture and helps to pelletise the suspended particles.

A centrifuge is provided. The centrifuge comprises: a centrifuge housing; a centrifuge rotor rotationally mounted in the centrifuge housing to rotate about a centrifuge axis; and a centrifuge container as discussed herein attached to the centrifuge rotor. This centrifuge takes advantage of the benefits of the centrifuge container.

The centrifuge may further comprise a plurality of centrifuge containers as discussed herein attached to the centrifuge rotor, each centrifuge container rotationally spaced about the centrifuge axis from one another. This increases the processing capacity of the centrifuge.

A method of operating the centrifuge is provided. The method comprises the steps of: flowing a liquid containing suspended particles into the centrifuge container; rotating the rotor at a first rotational speed to concentrate the suspended particles at an outer end of the centrifuge container as a concentrated pellet; rotating the rotor at a second rotational speed and removing the liquid from the centrifuge container; and flushing the concentrated pellet from the centrifuge container. This method effectively produces both a clarified liquid (supernatant) as well as a pellet of the suspended particles. Either or both can then be taken for further processing.

The centrifuge container used in any of the methods discussed herein may be the centrifuge container discussed herein. This takes advantage of the benefits of the method and the benefits of the centrifuge container. The centrifuge container is particularly beneficial for the method discussed herein.

## Brief Description of the Drawings

The present specification makes reference to the accompanying drawings, by way of example only, in which:

Figure 1 shows a schematic cross-section of a centrifuge container during a method of clarifying a liquid;
Figure 2 shows a schematic cross-section of the centrifuge container of Figure 1 at a later point during the method of clarifying a liquid;
Figure 3 shows a schematic cross-section of the centrifuge container of Figure 1 at an even later point during the method of clarifying a liquid;
Figure 4 shows a flow-chart of a method of clarifying a liquid; and
Figure 5 shows a flow-chart of a further method of clarifying a liquid.

## Detailed Description

Figures 1 to 3 show a centrifuge container 100. This centrifuge container 100 may be used in the method shown in Figures 4 and 5, but it also may be used in any other suitable centrifuge method.

The centrifuge container 100 is suitable for clarifying a liquid containing suspended particles 2. The centrifuge container 100 may be mounted in a centrifuge in order to achieve this. Specifically, the centrifuge container 100 may be attached to a centrifuge rotor which is rotationally mounted in a centrifuge housing. In use, the centrifuge rotor rotates about a central axis of rotation (identified as a centrifuge axis), thereby rotating the centrifuge container 100.

The centrifuge container 100 has a connection end 12 which is the end which, in use, connects to the centrifuge rotor. For example, this may be via a screw thread arrangement, an interference fit, a locking arrangement or any other suitable connection. The centrifuge container 100 further has an outer end 14. The outer end 14 is spaced from the connection end 12. Specifically, this spacing is along a longitudinal axis L of the centrifuge container 100 in a first direction. A second direction can be defined transverse (such as perpendicular) to the first direction), for example along a radial axis R. The longitudinal axis L is an axis extending centrally along the longest dimension of the centrifuge container 100. The first direction is from the connection end 12 to the outer end 14. In use, this first direction is radially outward from the centrifuge axis. This outer end 14 is where particles 2 are concentrated in use.

The centrifuge container 100 further comprises a number of fluid ports 22, 24, 26. This includes a first fluid port 22 which is for flowing of a liquid containing suspended particles 2 into the centrifuge container 100. The centrifuge container 100 further comprises a second fluid port 24 for flowing of a liquid containing suspended particles 2 from the centrifuge container 100. In certain operating modes, which will be described in more detail below, the liquid containing suspended particles 2 may recirculated through the centrifuge container by flowing out of the second fluid port 24 and back into the first fluid port 22.

The first fluid port 22 may include a declined section 34 which extends towards an inner wall of the centrifuge container 100. That is, the declined section 34 may be transverse to the first direction (i.e. the longitudinal axis) and/or to the second direction. As shown in Figures 1 to 3, the declined section 34 may have a curve.

A flushing tube 27 extends into the centrifuge container 100. A flushing port 26 can be identified at or near to the connection end 12. The flushing tube 27 extends from the connection end 12 towards the outer end 14. That is, along the first direction. In certain examples, this flushing tube 27 may be co-axial (aligned with) with the longitudinal axis L of the centrifuge container 100. Additionally, or alternatively, the flushing tube 27 may be arranged centrally in the centrifuge container 100. The flushing tube 27 is spaced from the outer end 14 to allow flushing fluid to enter the centrifuge container 100 via the flushing tube 27. The flushing tube 27 allows for delivery of a flushing fluid to the centrifuge container 100 in order to flush concentrated particles from the centrifuge container 100. The flushing tube 27 includes an outlet 32 which will be discussed in more detail below.

The first fluid port 22, second fluid port 24, and flushing tube 27 may be spaced from one another in the second direction. That is, the first fluid port 22, second fluid port 24, and flushing tube 27 (specifically the flushing port 26) may be arranged in a line/plane with one another. For example, the flushing tube 27 may be between the first fluid port 22 and the second fluid port 24 in this second direction. In use, such as when attached to the centrifuge rotor, the first fluid port 22 may be below the flushing tube 27 and/or below the second fluid port 24.

The centrifuge container 100 has an inner surface and an outer surface. One or both of these may be rotationally symmetric about the longitudinal axis L of the centrifuge container 100.

The centrifuge container 100 may be generally tapering in shape. That is, the centrifuge container 100 may taper in the first direction. The taper means that a cross-section of the centrifuge container 100 reduces in area. This does not necessarily mean that the entire centrifuge container 100 is tapered. For example, the centrifuge container 100 may be generally a cylindro-conical shape. That is, there may be a tapering section and a non-tapering section. The non-tapering section may, for example, be generally cylindrical. The non-tapering section may be closer to the connection end 12, with the tapering section extending from the non-tapering section to the outer end 14. The tapering section may be longer than the non-tapering section. For example, the tapering section may be at least twice or four times as long as the cylindrical section.

The taper of the centrifuge container 100 can be any suitable angle. Specifically, a taper angle $\alpha$ may be selected such that fluid flow in the centrifuge container 100 from the outer end 14 to the connection end 12 does not experience flow separation. That is, the boundary layer does not detach from the inner surface of the centrifuge container 100. This could be tested, for example, by monitoring the separation quality of the resulting product. Additionally, or alternatively, a transparent centrifuge container 100 could be used and the flow separation could then be observed. Different taper angles could be tested until there is no flow separation (or flow separation below an acceptable threshold) observed.

For example, the taper may have a taper angle $\alpha$ (full taper) of 30 degrees or less, such as 16 degrees or less, or 10 degrees or less. The taper angle $\alpha$ may be at least 3 degrees a specific taper angle $\alpha$ may be 4 degrees. A total opening angle may be 8 degrees, that is the sum of the two individual taper angles

The outer end 14 of the centrifuge container may be shaped to encourage pelleting of the suspended particles 2 and/or to encourage fluid flow through the centrifuge container 100. For example, the outer end 14 of the centrifuge container may be at least partially shaped as a torus. That is, the outer end 14 of the centrifuge container may be at least partially toroidal in shape. A torus is a surface of revolution generated by revolving a circle in three-dimensional space one full revolution about a torus axis that is coplanar with the circle. In the present example, the torus axis may be co-axial with the longitudinal axis L. This shape effectively re-directs fluid flow while maintain momentum and minimising pressure loss.

Figures 1 to 3 show the outer end 14 shaped as a semi-torus which is one suitable shape for the outer end 14. The semi-torus is formed by splitting a torus in half along a plane which is perpendicular to the torus axis. In other words, the plane passes through a centre of the circle used to define the torus about the circle's entire revolution about the torus axis.

The torus used to shape the outer end 14 can be any type of torus, including a ring torus, a horn torus, or a spindle torus. In certain examples, the torus may specifically be a horn torus. A horn torus being one where the torus axis of revolution is tangent to the circle used to define the torus.

A centre of the torus can be defined as an intersection between the torus axis and a plane which passes through a centre of the circle used to define the torus about the circle's entire revolution about the torus axis. The centre of the torus may be aligned, such as co-axial, with the longitudinal axis L and/or the outlet 32 of the flushing tube 27. In certain examples, the centre of the torus may be adjacent to the outlet 32 of the flushing tube 27. That is, they may be immediately next to one another - such as within 10 millimetres. In certain examples the outlet 32 of the flushing tube 27 and the centre of the torus may be co-planar with one another.

With the torus used to define the shape of the outer end 14, a major diameter and a minor diameter can be defined. The major diameter is the total diameter of the torus across its entire width. In other words, the distance between outermost edges of the circle when it is 180° apart in the revolution used to define the torus (or twice the distance between the outermost edge of the circle and the torus axis). The minor diameter is the diameter between the centre of the circle when it is 180° apart in the revolution used to define the torus (or twice the distance between the centre of the circle and the torus axis).

The minor diameter of the torus may be at least 0.5 times a diameter of the flushing tube, preferably at least 1 times, more preferably at least 1.5 times.

Separately, or additionally, the major diameter of the torus may be at least 1 times a diameter of the flushing tube, preferably at least 1.5 times, more preferably at least 2 times.

An inner diameter of the torus can also be defined as the distance between innermost edges of the circle when it is 180° apart in the revolution used to define the torus (or twice the distance between the innermost edge of the circle and the torus axis). For a horn torus the inner diameter is 0, and for a spindle torus the inner diameter is negative.

A radius of the circle used to define the torus may also be defined. This is selected as appropriate for the desired size of the torus. For example, the radius of the circle used to define the torus may be substantially the same as a radius of the flushing tube 27. In this context, substantially the same may be within 25%, within 10%, or within 5%.

In use, the centrifuge container 100 is attached to a centrifuge rotor inside a centrifuge housing. Typically, multiple centrifuge containers 100 would be attached to the centrifuge rotor. Then, the rotor is spun to operate the centrifuge.

A method of clarifying a liquid containing suspended particles 2 will now be described with reference to Figures 4 and 5. The liquid can be identified as a supernatant. The suspended particles 2 may be one or more of cell debris, proteins, bacteria, etc.. In general the suspended particles 2 may have a size of 50μm or less. In specific examples this may be 10μm or less, or 1μm or less, or even 0.1μm or less.

This method can be performed using the centrifuge container 100 of Figures 1 to 3 and so some reference will be made to features of this specific centrifuge container 100. However, this must not be taken as limiting. The method can be applied to any suitable centrifuge container 100 and not just those as shown in Figures 1 to 3.

In a first step 102, a liquid containing suspended particles 2 is flowed into a centrifuge container 100 which is arranged on a centrifuge rotor. This may the be centrifuge container 100 of Figures 1 to 3, or any other centrifuge container 100. The flowing of the liquid containing suspended particles 2 could be via use of a pump operating in a first direction.

The rotor is then rotated at a first rotation speed in step 103. This concentrates the suspended particles 2 at an outer end 14 of the centrifuge container 100. The concentrated particles form a concentrated pellet. This first rotational speed may be in a first direction (such as clockwise or anti-clockwise). This may generally be a maximum operating speed for the centrifuge rotor. That is, the highest speed that the centrifuge rotor spins during the process. This could be defined by the process and/or by a maximum safe speed for the particular apparatus. The first rotational speed may be sufficient to induce 10,000 xg to 25,000 xg. For example, the first rotational speed may be sufficient to induce 20,000 xg. In further examples, the first rotational speed may be up to 100,000 xg.

Figure 1 shows steps 102 and 103 for the centrifuge container 100 of Figures 1 to 3. For this centrifuge container 100 the liquid containing suspended particles is flowed in via the first fluid port 22. However, the flow may be via any suitable fluid port.

The pellet of suspended particles 2 is formed in the outer end 14. For example, this may be within the torus-shaped region of the centrifuge container 100 of Figures 1 to 3.

In certain examples (such as shown in Figure 5), the liquid containing suspended particles 2 is recirculated through the centrifuge container 100 in steps 104, 105. That is, it is flowed from the centrifuge container 100 (step 104) and then flowed back into the centrifuge container (step 105).

For the centrifuge container 100 of Figures 1 to 3, the flow from the centrifuge container 100 (step 104) may be via the second fluid port 24, and then returned back to the centrifuge container 100 (step 105) by the first fluid port 24.

During this recirculation the centrifuge rotor may be rotated at a rotational speed (such as a third rotational speed). This third rotational speed may be the same as the first rotational speed.

A particle detector may be provided to detect an amount of suspended particles 2 in the liquid flowing from the centrifuge container 100 (step 104) and/or into the centrifuge container 100 (step 105). This may be one or more individual detectors. The detectors may be able to quantify the amount of suspended particles 2 in the liquid, or may simply be a binary indicator of whether there are suspended particles 2 present or not. Example particle detectors include turbidity sensors, particle size sensors, particle counters, etc..

The recirculation may be continued as long as the particle detector indicates that an amount of suspended particles 2 in the liquid being recirculated is greater than a threshold. This threshold may be at or near to 0, or may be set at an acceptable level. In such cases, it is only when the amount of suspended particles 2 in the recirculated flow is below the threshold that the method can continue on to further steps.

Additionally, or alternatively, the method may be prevented from continuing until an estimated settling time for the suspended particles 2 has passed. This settling time may be estimated based on one or more of: physical properties of the suspended particles; dimensions of the centrifuge container; physical properties of the liquid; and/or the first rotational speed.

In a specific example, the settling velocity ($v_p$) for a suspended particle 2 (such as in the centrifuge container 100 of Figures 1 to 3) may be found by the Stokes equation as:

$$v_p = \frac{2}{9}\frac{r^2 g(\rho_p - \rho_f)}{\eta} \quad \text{(Equation 1)}$$

Where r is the radius of the suspended particle 2. The density of the particle is $\rho_p$ and the density of the fluid is $\rho_f$. g is gravitational acceleration. The viscosity of the fluid is $\eta$.

With this settling velocity ($v_p$), the settling time ($t_s$) can then be calculated based on a length ($l_c$) of the centrifuge container 100. For example, a length of the centrifuge container 100 from connection end 12 to outer end 14. This settling time ($t_s$) can be calculated as:

$$t_s = \frac{l_c}{v_p} \quad \text{(Equation 2)}$$

With this settling time calculated, the steps 103 (and, where appropriate, 104 and 105) can be repeated until at least the settling time has passed. There may be a buffer such that the actual time waited is slightly longer than the calculated settling time (such as by 10% extra) to allow for any variations in the actual time required.

The method further comprises a draining step. This can encompass steps 107 and 108 shown in Figures 4 and 5. The centrifuge rotor is rotated at a second rotational speed (step 107) and the liquid is removed from the centrifuge container 100. This leaves the suspended particles 1 in the pellet at the outer end 14. The liquid may be removed by applying a vacuum and sucking the liquid from the centrifuge container 100.

The second rotational speed may be in a different direction to the first rotational speed. For example, if the first rotational speed is in a clockwise direction then the second rotational speed may be in a counter-clockwise direction, or vice-versa. The method may take advantage of the Coriolis forces in order to aid concentration and/or recovery. When the direction of fluid flow is changed the direction of rotation needs to also be reversed in order to take advantage of these Coriolis forces.

The second rotational speed is selected as an appropriate speed to drain the centrifuge container 100. In certain examples, this may be slower than the first rotational speed. Noting that the first rotational speed and second rotational speed may be in different directions, it is the magnitude (or absolute value) of each that is compared for this comparison. In certain examples, the first rotational speed may be at least twice the second rotational speed, or at least five times, or at least 10 times. The second rotational speed may be, for example, less than 500 rpm, such as between 300 rpm and 500 rpm.

A gas, such as air or any other suitable gas, may be flowed into the centrifuge container 100 to replace the liquid. In certain examples, the gas may be pumped to create a pressure to encourage the liquid to exit the centrifuge container 100. This could be achieved, by example, by removing material from the centrifuge container 100 (such as via a pump) to thereby generate a negative pressure. A pump could be operated in a second direction, opposite to the first direction of the pump, to remove material from the centrifuge container 100. In other words, and operating direction of the pump can be reversed.

Figure 2 shows this draining step for the centrifuge container 100. However, as noted the method is not restricted to the centrifuge container 100 of Figures 1 to 3. The liquid may be removed from the centrifuge container 100 via the first fluid port 22. The declined section 34 of the first fluid port 22 may be particularly beneficial to remove more liquid from the centrifuge container 100. The gas may enter through the second fluid port 24.

The next step of the method is a recovery step (step 109). In this step, the concentrated pellet is removed from the centrifuge container 100. For example, a flushing fluid may be delivered to the centrifuge container 100 in order to flush the concentrated pellet from the centrifuge container. The flushing fluid can dissolve the concentrated pellet, and/or mechanically displace the pellet. The flushed pellet can then be recovered in any suitable method.

The flushing fluid may be any suitable fluid. In certain examples this may be one or more of: a saline solution; buffer solution; de-ionized water; water for injection; and/or phosphatebuffered saline. The flushing fluid may be selected based on the intended further processing of the pellet. For example, if the pellet is waste material which will not be subject to further processing then de-ionized water may be used. If the pellet is a product of interest for collection and/or further processing then the flushing fluid may be a buffer which is neutral to the suspended particles 2 - for example water for injection; and/or phosphatebuffered saline. The flushing fluid could also be a fluid which acts to loosen the pellet so as to facilitate resuspension of the particles.

In certain cases, steps 108 and 109 could be (at least partially) combined. That is, the liquid could be removed from the centrifuge container 100 at the same time that the pellet is removed. In order to achieve this, the method may comprise controlling a rate of flow from the centrifuge container 100 via one or both fluid flow lines - a supernatant flow line and a pellet flow line. The supernatant flow line may be via the first fluid port 22. The pellet flow line may be via the flushing port 26.

Figure 3 shows this flushing step for the centrifuge container 100 of Figures 1 to 3. The flushing fluid may be flowed into the centrifuge container 100 via the flushing tube 27. The flushing tube 27 extending to the outer end 14 of this centrifuge container 100 helps to direct the flushing fluid to the pellet. The pellet can then exit the centrifuge container 100 by any of the other fluid ports 22, 24. Figure 3 shows the pellet exiting via the first fluid port 22.

In certain examples, the centrifuge container 100 (either that of Figures 1 to 3, or any other suitable centrifuge container 100) may be removed form the centrifuge rotor. In some examples, this could be done instead of flushing in order to recover the pellet. A new centrifuge container 100 can then be attached to the centrifuge rotor and the process can be repeated. In this sense, each centrifuge container 100 can be generally a single-use component for one cycle of clarifying a liquid.

As noted above, the method of Figure 5 is generally the same as the method of Figure 4 but it further includes the recirculation steps 104 and 105. Any disclosure in relation to the method of Figure 4 is equally applicable to this method of Figure 5, and vice-versa.

This method can be operated in both a closed loop mode or a batch mode. This may depend on the volume to be processed. If the processed volume equals the chamber volume, batch mode is possible. If the processed volume exceeds chamber volume, continuous flow or separated batches are possible. The chamber volume is the sum of the internal volumes of all centrifuge containers 100.

Figures 6 to 6G show operation of a system 1000 comprising a centrifuge container 100 in an open loop, along with an option to recover the pellet. After a first run has been performed, a second run could be performed (in a batch mode) This would particularly be useful if the amount of suspended particles 2 and hence pellet is too great for the volume of the system 1000 (specifically the volume of the centrifuge rotor and container(s) 100).

Figures 7 to 7G show operation of a system 1000 comprising a centrifuge container 100 in a closed loop mode, which can allow for continuous operation of the system 1000 regardless of the volume of the system 1000 (specifically the volume of the centrifuge rotor and container(s) 100).

The centrifuge container 100 may generally be as discussed herein, including any of the modifications discussed. These Figures show a single centrifuge container 100, but it is appreciated that there may be a plurality of connected centrifuge containers 100. The Figures also do not show the rotor or centrifuge since they are schematic.

The systems 1000 include slight constructional differences, but it is appreciated that in general either mode of operation may be performed on each system 1000. If an additional valve or line needs to be added then this may be included. For the avoidance of doubt, any disclosure in relation to the system 1000 of Figures 6 to 6G is equally applicable to the system of Figures 7 to 7F, or vice-versa.

A large number of valves are shown in the systems 1000. In general, when the valve symbols are filled with a solid black colour they are in a closed position in which flow through the valve is prevented or inhibited. When the valve symbols are filled with white they are in an open position in which flow through the valve is permitted. It is noted that the exact provision of valves may be varied and the Figures show an example of suitable valving while others are equally applicable. What will be understood is that the relevant disclosure is of the various flow paths between components and that these flow paths can be executed using any arrangement of fluid lines and valves.

The system 1000 includes a rinse reservoir 70. Either side of the rinse reservoir 70 may be a rinse inlet valve 74 and a rinse outlet valve 76. The rinse inlet valve 74 selectively controls a flow of fluid into the rinse reservoir 70. The rinse outlet valve 76 selectively controls a flow of fluid out of the rinse reservoir 70. In use, this rinse reservoir 70 has a supply of fluid, such as a buffer fluid. This fluid may form the supernatant fluid.

The system 1000 may further include a bioreactor 60. Either side of the bioreactor 60 may be a bioreactor inlet valve 64 and a bioreactor outlet valve 66. The bioreactor inlet valve 64 selectively controls a flow of fluid into the bioreactor 60. The bioreactor outlet valve 66 selectively controls a flow of fluid out of the bioreactor 60. In use, the bioreactor 60 may be operated to produce the suspended particles 2. In alterative arrangements, the suspended particles 2 may be produced separately and provided into the system 1000.

The system 1000 further includes a pellet container 80, for recovering of the flushed pellet. A pellet valve 84 selectively controls flow into the pellet container 80. There may also be a pellet sensor 82 which provides information regarding an amount of material in the pellet container 80. For example, the pellet sensor 82 may include one or more of a fluid level sensor, a weight sensor, or any other suitable sensor.

The system 1000 further includes a supernatant container 90, for recovering of supernatant. A supernatant valve 94 selectively controls flow into the supernatant container 90. There may also be a supernatant sensor 92 which provides information regarding an amount of material in the supernatant container 90. For example, the supernatant sensor 92 may include one or more of a fluid level sensor, a weight sensor, or any other suitable sensor.

The system 1000 may further comprise an air inlet 59 for inletting air into the system 1000. For example, this could include a pump. An air inlet valve 58 connects this air inlet 59 to the rest of the system 1000 and selectively permits or inhibits flow from the air inlet 59 to the rest of the system 1000.

The system 1000 can also comprise a number of sensors. In examples one or more of these sensors can be omitted. The system of Figure 6 includes an air/liquid sensor 51, a pressure sensor 52, a flow sensor 56, and a turbidity sensor 57. Any suitable combination of one or more of these sensors may be considered.

The system 1000 comprises a pump 50 which is suitable for driving the liquid with suspended particles 2 through the system 1000. In certain examples, the air/liquid sensor 51 and the pressure sensor 52 may be provided either side of the pump 50. The pump 50 may be a reversible pump.

The system 1000 may also comprise a bypass valve 54 which connects the pump 50 to the pellet container 80 and/or the supernatant container 90. The system 1000 may further comprise a loop valve 55, which can be opened in order to operate the system 1000 in a closed loop. That is, output from the centrifuge container 100 may be returned thereto.

A first valve 41, second valve 42, and third valve 44 are used to selectively direct fluid flow into the various fluid ports 22, 24, 26 of the centrifuge container. Collectively, this may be referred to as valving and the exact arrangement of valves can be modified accordingly. A flushing port valve 126 may be provided to selectively inhibit or allow flow to the flushing port 26. In certain examples there may be a first port valve to selectively inhibit or allow flow to the first fluid port 22. There may, additionally or alternatively, be a second port valve to selectively inhibit or allow flow to the second fluid port 24.

Operation of the system 1000 of Figure 6 to clarify a liquid containing suspended particles 2 will now be described with respect to Figures 6A to 6G. While the precise valve combinations will be noted with respect to the Figures, it is fully appreciated that alternative valving arrangements could still deliver the same fluid flows so as to perform this operation. Equally, the operation may be modified to not include any components which are not expressly set out as required. Unless specified otherwise, each valve is in a closed position in which fluid flow therethrough is prevented/inhibited.

Figure 6A shows a step in which static filling of the centrifuge container 100 is performed. In certain examples, this step may be omitted. The rinse inlet valve 74 and rinse outlet valve 76 are open. The pump 50 is operating in a first direction. The first valve 41 is open.

The second valve 43 is closed. The third valve 44 is open. Fluid from the riser reservoir 70 is pumped by the pump 50 into the centrifuge container 100. In Figure 6A this is via the first fluid port 22, but it could equally be via the flushing port 26 or the second fluid port 24. This will then fill up the centrifuge container 100. Once the centrifuge container 100 is suitably filled then the fluid will leave via the second fluid port 24, and be returned back to the rinse reservoir 70. This step can be continued until the flow sensor 56 indicates a flow of fluid from the centrifuge container 100. This can indicate that the centrifuge container 100 has been suitably filled. In this operation, the pump 50 may be operated at a constant speed and/or a constant pumping volume. In this step, the centrifuge is not operating and so the centrifuge container 100 is not being rotated.

The centrifuge may then begin to operate to rotate the centrifuge container 100, as shown in Figure 6B. This rotation may be at a first rotational speed as described herein. For example, the first rotational speed may be in the region of 200 to 400 rotations per minute (rpm), such as 300 rpm. The pump 50 continues to pump fluid from the rinse reservoir 70 to the centrifuge container. This step can be continued until the flow sensors 56 indicates a flow of fluid from the centrifuge container 100. The overall flow paths are generally the same as in Figure 6A, but with the centrifuge container 100 being rotated.

Figure 6C then shows the suspended particles 2 being introduced to the centrifuge container 100. In this example, the suspended particles 2 flow from the bioreactor 60 but as discussed herein they can be provided from any other suitable source. Figure 6C shows the bioreactor inlet valve 64 and bioreactor outlet valve 66 being opened. The rinse inlet valve 74 and rinse outlet valve 76 are closed. The centrifuge container 100 continues to be rotated by the rotor, for example at a first rotational speed. This may be the same speed, or a different speed, to the speed of Figure 6B.

The liquid containing suspended particles 2 is thus flowed into the centrifuge container 100. The rotation of the centrifuge container 100 acts to concentrate the suspended particles at an outer end 14 of the centrifuge container 100. in certain examples, the pump 50 may be controlled passed on a reading from the flow sensor 56 to set a desired flow rate of liquid containing suspended particles 2. This step can be repeated until the turbidity sensor 57 indicates that the turbidity has dropped below a lower threshold. This indicates that the suspended particles 2 are being separated from the liquid.

Figure 6D shows the next step in the method. The bioreactor inlet valve 64 is closed, and the supernatant valve 94 is opened. This is to recover the supernatant in the supernatant container 90. This can be performed until a maximum capacity of the system 1000 has been reached (for example based on how much volume all of the centrifuge containers 100 in the system can hold), or until the bioreactor 60 is empty. This then leaves the pellet in the centrifuge container 100 for recovery.

In certain examples it may be beneficial to suck out any leftover supernatant from the centrifuge container. Such a step is shown in Figure 6E. The bioreactor outlet valve 66 is closed and the bypass valve 54 is opened. The third valve 44 is closed and the air inlet valve 58 is opened. The pump 50 is then operated in an opposite direction which draws fluid out of the centrifuge container 100, such as through the first fluid port 22. This creates a negative pressure in the centrifuge container 100 such that air is drawn in through the air inlet 59 and second fluid port 24. in certain examples, the air may be pumped into the centrifuge container 100. The supernatant is therefore drawn out from the centrifuge container, via the first fluid port 22 and then through the bypass valve 54 to the supernatant container 90.

The air/liquid sensor 51 is provided such that it senses this bypass flow. Once the air/liquid sensor 51 detects air this means that the centrifuge container has been substantially drained of supernatant and this step can then be finished.

Recovery of the pellet of suspended particles 2 can then be performed. The rinse outlet valve 76 is opened. The bypass valve 54 is closed. The second valve 43 is opened. The air inlet valve 58 is closed. The flushing port valve 126 is opened. The supernatant valve 94 is closed. The pellet valve 84 is opened. The pump 50 is reversed again such that it delivers rinsing fluid via the flushing port 26 to flush the pellet from the centrifuge container 100. in this step the centrifuge container 100 can be rotated at the second rotational speed as discussed herein.

The pelletised suspended particles 2 are then recovered via the first fluid port 22 to the pellet container 80. This step can be continued until the turbidity sensor 57 indicates that the turbidity has fallen below a threshold, and/or is reducing. This indicates that there are not suspended particles 2 being delivered to the pellet container 80 but instead just rinsing fluid.

A further recovery step can additionally or alternatively be performed, as shown in Figure 6G. The air inlet valve 58 is opened. The rinse outlet valve 78 is closed. The flushing port valve 126 is closed. The first valve 41 is opened and the second valve 43 is closed. The bypass valve 54 is opened. Again, a negative pressure is generated in the centrifuge container 100 which draws air in via the air inlet 59. Any remaining rinse fluid and pellet of suspended particles 2 is removed via the first fluid port 22 and travels via the bypass valve 54 to the pellet container 80.

Again, the air/liquid sensor 51 is provided such that it senses this bypass flow. Once the air/liquid sensor 51 detects air this means that the centrifuge container has been substantially drained of rinse liquid and/or pellet and this step can then be finished.

This therefore shows a first mode of operation of the system 1000.

A further system 1000 is shown in Figures 7 to 7G. Unless otherwise expressly stated otherwise this system 1000 is the same as the system 1000 of Figures 6 to 6G. Any disclosure in relation to the system 1000 of Figures 7 to 7G is equally applicable to the system 1000 of Figures 6 to 6G, and vice-versa.

While Figures 7 to 7G show the bioreactor inlet valve 64 it is noted that this may not be present in all examples. In some examples, there may be no inlet line to the bioreactor 60 in the system 1000, and hence no bioreactor inlet valve 64. In such cases the bioreactor 60 may be separately suppled with feed material.

The system 1000 may further comprise a waste container 96. A waste valve 95 selectively controls flow into the waste container 96. The flushing port valve 126 connects to between the second valve 43 and the third valve 44. A fourth valve 45 is provided between the bypass valve 54 and this connection.

A variable restriction 53a may be provided between the bypass valve 54/fourth valve 45 and a top fluid line of the system 1000. In further examples, this variable restriction 53a may be omitted.

The top fluid line is in fluid communication with one or more of: the pellet container 80; the supernatant container 90; the bioreactor 60, the rinse reservoir 70, and/or the waste container 96. Specifically, it may be the inlet to each of these components. The variable restriction 53a can be used to control a rate of flow of fluid. This variable restriction 53a may be omitted in certain examples.

A fifth valve 46 may be provided between the second fluid inlet 22 and the various components provided on the top fluid line of the system 1000. A sixth valve 48 is provided between the pellet container 80 and supernatant container 90 on this top fluid line. In this example, there may be no inlet to the bioreactor 60 and hence no bioreactor inlet valve 44. The air/liquid sensor 51 may be omitted.

A connection mat be provided between the flushing port 26 and the top fluid line. This connection may be via a variable restriction 53. The variable restriction 53 can be used to control a rate of flow of fluid. The flushing fluid port 26 may also be connected to this top fluid line. Along this connection there may be a flow sensor 56a and/or a turbidity sensor 57a.

Operation of the system 1000 of Figure 7 to clarify a liquid containing suspended particles 2 will now be described with respect to Figures 7A to 7G. While the precise valve combinations will be noted with respect to the Figures, it is fully appreciated that alternative valving arrangements could still deliver the same fluid flows so as to perform this operation. Equally, the operation may be modified to not include any components which are not expressly set out as required. Unless specified otherwise, each valve is in a closed position in which fluid flow therethrough is prevented/inhibited.

Figure 7A shows a filling step for the centrifuge container 100. The centrifuge container may be rotated during this step, for example at between 500 to 1500 rpm, such as at 1000 rpm. The rinse inlet valve 74 and rinse outlet valve 76 are open. The first valve 41 and second valve 43 are open. The flushing valve 126 is open. The fifth valve 46 is open.

The pump 50 is operated in a first direction such that fluid from the rinse reservoir 70 is supplied to the centrifuge container 100 via the flushing tube 27 (and/or via the first fluid port 22).

Figure 7B then shows a second filling step, which can be performed in addition to or as an alternative to the step of Figure 7A. The flushing valve 126 and second valve 43 are close. The pump 50 is continued to operate in the first direction such that fluid from the rinse reservoir 70 is supplied to the centrifuge container 100 via the first port 22. The centrifuge container 100 may again be rotated, for example at the same speed as in Figure 7A.

Step 7C then shows the suspended particles 2 being introduced to the centrifuge container 100. The bioreactor valve 66 is opened and hence the pump 50 delivers the suspended particles 2 to the first port 22 and hence into the centrifuge container 100. The second valve 44 is opened, as is the fourth valve 45. The fifth valve 46 is closed. The supernatant valve 94 is opened. The pellet valve 84 is opened.

A rotational speed of the centrifuge container 100 may be increased, for example to a first rotational speed or to a second rotational speed as discussed herein. This may be, for example, at a maximum speed such as 6000 rpm or greater. For example, this may be at approximately 8000 rpm. This could also be defined with respect to g-force applied during the rotation. The small pellet (typically between 1 $\mu$m and 4 $\mu$m) along with a small density difference between the fluid and the suspended particles 2 means that high g-forces may be required. For example, the g-forces may be 5000 or greater, preferably 10,000 or greater, more preferably 20,000 or greater.

The (or each, depending on how many are present) variable restrictions 53, 53a can then be adjusted to control their respective flow rates in order to allow for separation of the pellet of suspended particles 2 from the supernatant. Supernatant flows through the second port 24, second valve 44, fourth valve 45 and then the variable restriction 53a (when present) and subsequently into the supernatant container 90.

The pellet of suspended particles 2 flows through the flushing tube 27, the variable restriction 53 and into the pellet container 80. The or each variable restriction 53a may be controlled based on readings from the or each turbidity sensor 57, 57a. The turbidity sensor 57 which senses the returned supernatant is compared to a lower threshold value and when the sensed value is above this then operation of the system 1000 may be modified. The turbidity sensor 57a of the pellet collection may be compared to a higher threshold value and if the sensed value is below this threshold then operation of the system 1000 may be modified.

This step may be ended when there is no more flow in the supernatant return from the second port 24, such as determined by the flow sensor 56.

Figure 7D shows how rotation of the centrifuge container 100 can then force out the rest of the pellet of suspended particles 2. The air inlet valve 58 is opened, as are the second valve 43 and third valve 44. As a result, air is delivered to the centrifuge container 100 via the first fluid port 22 (and/or the second fluid port 24). The pellet is then removed via the flushing tube 27 and its outlet 32.

in this step, the centrifuge container 100 may be rotated, such as at a second rotational speed as discussed herein. This may be in an opposite direction to a rotational speed of earlier steps.

The system 1000 may be cleaned, such as shown in Figure 7E. This could be via fluid from the rinse reservoir 70, by opening the rinse outlet valve 76, or a separate fluid supply. The first valve 41 is opened. The second valve 43 and third valve 44 are closed. The air inlet valve 58 is closed. The waste valve 95 is opened. The pellet valve 84 is closed. The fifth valve 46 is opened.

The pump 50 acts to pump the rinse fluid into the centrifuge container 100 by the first fluid port 22. The centrifuge container 100 can be rotated in the centrifuge during this step. This may be in the same direction and/or speed as the first rotational speed and/or the second rotational speed. The rinse fluid then passes through the second port 24 and the fifth valve 46 to the waste container 96.

Figure 7F shows an additional, or alternative, cleaning step to Figure 7E. In this step, the rinse fluid is directed through the flushing port 26 and flushing tube 27 (and/or through the second fluid port 22) to rinse the centrifuge container 100. The flushing valve 126 is opened. The first valve 41 is opened. The centrifuge container 100 may be rotated during this step. This may be in the same direction and/or speed as the first rotational speed and/or the second rotational speed. The rinse fluid then passes through the second port 24 and the fifth valve 46 to the waste container 96.

Finally, Figure 7G shows an emptying of the system 1000. In this step, the air inlet valve 58 is opened. This allows air to enter the centrifuge container 100 via the second port 24. The second valve 43 and flushing valve 126 are closed. The first valve 41 is opened. The bypass valve 54 is opened. The pump 50 is operated in an opposite direction so as to drive any fluid from the centrifuge container 100 to the wase container 96, such as through the first valve 41 and the bypass valve 54.

In this sense, continuous clarification of the liquid containing suspended particles 2 can be performed. The steps can be repeated in order to clarify more liquid containing suspended particles 2.

It will be appreciated that embodiments of the disclosure may be implemented using a variety of different information processing systems. In particular, although the Figures and the discussion thereof provide exemplary computing systems and methods, these are presented merely to provide a useful reference in discussing various aspects of the disclosure. Embodiments may be carried out on any suitable data processing device, such as a personal computer, laptop, tablet, personal digital assistant, mobile telephone, smart phone, set top box, television, server computer, etc.. Of course, the description of the systems and methods has been simplified for purposes of discussion, and they are just one of many different types of systems and methods that may be used. It will be appreciated that the boundaries between logic blocks are merely illustrative and that alternative embodiments may merge logic blocks or elements, or may impose an alternate decomposition of functionality upon various logic blocks or elements.

It will be appreciated that the above-mentioned functionality may be implemented as one or more corresponding modules as hardware and/or software. For example, the above-mentioned functionality may be implemented as one or more software components for execution by a processor of the system. Alternatively, the above-mentioned functionality may be implemented as hardware, such as on one or more field-programmable-gate-arrays (FPGAs), and/or one or more application-specific-integrated-circuits (ASICs), and/or one or more digital-signal-processors (DSPs), and/or other hardware arrangements. Method steps implemented in flowcharts contained herein, or as described above, may each be implemented by corresponding respective modules. Moreover, multiple method steps implemented in flowcharts contained herein, or as described above, may be implemented together by a single module.

It will be appreciated that, insofar as embodiments of the disclosure are implemented by a computer program, then a storage medium and a transmission medium carrying the computer program form aspects of the disclosure. The computer program may have one or more program instructions, or program code, that, when executed by a computer, causes an embodiment of the disclosure to be carried out. The term *"program"* as used herein, may be a sequence of instructions designed for execution on a computer system, and may include a subroutine, a function, a procedure, a module, an object method, an object implementation, an executable application, an applet, a servlet, source code, object code, a shared library, a dynamic linked library, and/or other sequences of instructions designed for execution on a computer system. The storage medium may be a magnetic disc (such as a hard drive or a floppy disc), an optical disc (such as a CD-ROM, a DVD-ROM or a Blu-ray disc), or a memory (such as a ROM, a RAM, EEPROM, EPROM, Flash memory or a portable/removable memory device), etc.. The transmission medium may be a communications signal, a data broadcast, a communications link between two or more computers, etc..

Each feature disclosed in this specification, unless stated otherwise, may be replaced by alternative features serving the same, equivalent, or similar purpose. Thus, unless stated otherwise, each feature disclosed is one example only of a generic series of equivalent or similar features.

As used herein, including in the claims, unless the context indicates otherwise, singular forms of the terms herein are to be construed as including the plural form and, where the context allows, vice versa. For instance, unless the context indicates otherwise, a singular reference herein including in the claims, such as "a" or *"an"* (such as a component or an element) means *"one or more"* (for instance, one or more components, or one or more elements). Throughout the description and claims of this disclosure, the words *"comprise", "including", "having"* and *"contain"* and variations of the words, for example *"comprising"* and *"comprises"* or similar, mean that the described feature includes the additional features that follow, and are not intended to (and do not) exclude the presence of other components.

The use of any and all examples, or exemplary language (*"for instance", "such as", "for example"* and like language) provided herein, is intended merely to better illustrate the disclosure and does not indicate a limitation on the scope of the disclosure unless otherwise claimed. No language in the specification should be construed as indicating any non-claimed element as essential to the practice of the disclosure.

Any steps described in this specification may be performed in any order or simultaneously unless stated or the context requires otherwise. Moreover, where a step is described as being performed after a step, this does not preclude intervening steps being performed.

All of the aspects and/or features disclosed in this specification may be combined in any combination, except combinations where at least some of such features and/or steps are mutually exclusive. In particular, the preferred features of the disclosure are applicable to all aspects and embodiments of the disclosure and may be used in any combination. Likewise, features described in non-essential combinations may be used separately (not in combination).

A method of manufacturing and/or operating any of the devices disclosed herein is also provided. The method may comprise steps of providing each of the features disclosed and/or configuring or using the respective feature for its stated function.

CLAUSES:

1. A method of clarifying a liquid containing suspended particles, comprising the steps of:

   (a) flowing a liquid containing suspended particles into a centrifuge container arranged on a centrifuge rotor;
   (b) rotating the rotor at a first rotational speed to concentrate the suspended particles at an outer end of the centrifuge container as a concentrated pellet;
   (c) rotating the rotor at a second rotational speed and removing the liquid from the centrifuge container; and
   (d) flushing the concentrated pellet from the centrifuge container.

2. The method of clause 1, wherein the first rotational speed and the second rotational speed are in different directions to one another.

3. The method of any preceding clause, wherein the second rotational speed is slower than the first rotational speed.

4. The method of any preceding clause, wherein the first rotational speed is a maximum operating speed for the centrifuge rotor.

5. The method of any preceding clause, further comprising the step of:
recovering the flushed pellet.

6. The method of any preceding clause, wherein the liquid is flowed into the centrifuge container via a first fluid port in step (a) and the liquid is removed from the centrifuge container via the first fluid port in step (c).

7. The method of clause 6, wherein the concentrated pellet is flushed from the centrifuge container via the first fluid port.

8. The method of any preceding clause, further comprising between steps (b) and (c) the steps of:

(b-i) flowing the liquid from the centrifuge container; and
(b-ii) flowing the liquid back into the centrifuge container,

while rotating the rotor at a third rotational speed.

9. The method of clause 8, when dependent on clause 6, wherein the liquid is flowed from the centrifuge container via a second fluid port in step (b-i) and the liquid is flowed back into the centrifuge container via the first fluid port in step (b-ii).

10. The method of clause 8 or 9, wherein the third rotational speed is the same as the first rotational speed.

11. The method of any of clauses 8 to 10, further comprising the steps of:

detecting an amount of suspended particles in the liquid flowing from the centrifuge container and/or the liquid flowing into the centrifuge container;
repeating steps (b-i) and (b-ii) until the amount of suspended particles is below a threshold, and then proceeding to step (c).

12. The method of any preceding clause, further comprising the step of:

flowing a gas into the centrifuge container during step (c) and/or (d)

13. The method of clause 12, when dependent on clause 9, wherein the gas is flowed into the centrifuge container via the second fluid port.

14. The method of any preceding clause, further comprising the steps of:
estimating a settling time based on one or more of: physical properties of the suspended particles; dimensions of the centrifuge container; physical properties of the liquid; and/or the first rotational speed,
wherein step (b) is performed for the estimated settling time before step (c).

15. The method of any preceding clause, further comprising the steps of:

removing the centrifuge container from the centrifuge rotor;
attaching a new centrifuge container to the centrifuge rotor; and
repeating steps (a) to (d).

16. A centrifuge container for clarifying a liquid containing suspended particles, the centrifuge container comprising:

a connection end for connection to a centrifuge rotor;
an outer end for concentration of particles, the outer end spaced from the connection end in a first direction along a longitudinal axis of the centrifuge container;
a first fluid port at the connection end for flowing a liquid containing suspended particles into the centrifuge container;

a second fluid port at the connection end for flowing the liquid containing suspended particles from the centrifuge container; and
a flushing tube extending from the connection end towards the outer end, the flushing tube for delivering a flushing fluid to flush concentrated particles from the outer end.

17. The centrifuge container of clause 16, wherein the centrifuge container tapers in the first direction.

18. The centrifuge container of clause 17, wherein the taper angle is 30 degrees or less, preferably 16 degrees or less.

19. The centrifuge container of clause 17 or 18, wherein the taper angle is at least 3 degrees.

20. The centrifuge container of any of clauses 17 to 19, wherein the taper angle is such that fluid flow from the outer end to the connection end does not have flow separation.

21. The centrifuge container of any of clauses 16 to 20, wherein the centrifuge container comprises a cylindrical section at the connection end, followed by a tapering section which tapers in the first direction.

22. The centrifuge container of clause 21, wherein the cylindrical section has a shorter length in the first direction than the tapering section.

23. The centrifuge container of clause 22, wherein the tapering section is at least twice as long as the cylindrical section in the first direction, preferably at least four times as long.

24. The centrifuge container of any of clauses 16 to 23, wherein the outer end of the centrifuge container is at least partially shaped as a torus.

25. The centrifuge container of clause 24, wherein the outer end of the centrifuge container is a semi-torus.

26. The centrifuge container of clause 24 or 25, wherein the torus is a horn torus.

27. The centrifuge container of any of clauses 24 to 26, wherein a centre of the torus is aligned with an outlet of the flushing tube.

28. The centrifuge container of clause 27 wherein the outlet of the flushing tube is adjacent to the centre of the torus, preferably the outlet of the flushing tube is co-planar with the centre of the torus.

29. The centrifuge container of any of clauses 24 to 28, wherein the torus has a minor diameter of at least 0.5 times a diameter of the flushing tube, preferably at least 1 times, more preferably at least 1.5 times.

30. The centrifuge container of any of clauses 24 to 29, wherein the torus has a major diameter of at least 1 times a diameter of the flushing tube, preferably at least 1.5 times, more preferably at least 2 times.

31. The centrifuge container of any of clauses 16 to 30, wherein the first fluid port, the second fluid port and the flushing tube are spaced from one another in a second direction, transverse to the first direction.

32. The centrifuge container of clause 31, wherein the flushing tube is between the first fluid port and the second fluid port in the second direction.

33. The centrifuge container of clause 31 or 32, wherein the first fluid port comprises a declined section extending towards an inner wall of the centrifuge container.

34. The centrifuge container of any of clauses 16 to 33, wherein the flushing tube is aligned with the longitudinal axis of the centrifuge container.

35. The centrifuge container of any of clauses 16 to 34, wherein an inner surface and/or an outer surface of the centrifuge container is rotationally symmetric about the longitudinal axis of the centrifuge container.

36. A centrifuge comprising:

a centrifuge housing;
a centrifuge rotor rotationally mounted in the centrifuge housing to rotate about a centrifuge axis; and
a centrifuge container according to any of clauses 16 to 35 attached to the centrifuge rotor.

37. The centrifuge of clause 36, further comprising a plurality of centrifuge containers according to any of clauses 16 to 35 attached to the centrifuge rotor, each centrifuge container rotationally spaced about the centrifuge axis from one another.

38. A method of operating the centrifuge of clause 36 or 37, comprising the steps of:

flowing a liquid containing suspended particles into the centrifuge container;
rotating the rotor at a first rotational speed to concentrate the suspended particles at an outer end of the centrifuge container as a concentrated pellet;
rotating the rotor at a second rotational speed and removing the liquid from the centrifuge container; and
flushing the concentrated pellet from the centrifuge container.

39. The method of any of clauses 1 to 15, wherein the centrifuge container is according to any of clauses 16 to 35.

## Claims

**1.** A centrifuge container for clarifying a liquid containing suspended particles, the centrifuge container comprising:

a connection end for connection to a centrifuge rotor;
an outer end for concentration of particles, the outer end spaced from the connection end in a first direction along a longitudinal axis of the centrifuge container;
a first fluid port at the connection end for flowing a liquid containing suspended particles into the centrifuge container;
a second fluid port at the connection end for flowing the liquid containing suspended particles from the centrifuge container; and
a flushing tube extending from the connection end towards the outer end, the flushing tube for delivering a flushing fluid to flush concentrated particles from the outer end.

**2.** The centrifuge container of claim 1, wherein the centrifuge container tapers in the first direction,
preferably:

the taper angle is 30 degrees or less, preferably 16 degrees or less; and/or
the taper angle is at least 3 degrees.

**3.** The centrifuge container of claim 2, wherein the taper angle is such that fluid flow from the outer end to the connection end does not have flow separation.

**4.** The centrifuge container of any preceding claim, wherein the centrifuge container comprises a cylindrical section at the connection end, followed by a tapering section which tapers in the first direction,

preferably the cylindrical section has a shorter length in the first direction than the tapering section,
more preferably the tapering section is at least twice as long as the cylindrical section in the first direction, preferably at least four times as long.

**5.** The centrifuge container of any preceding claim, wherein the outer end of the centrifuge container is at least partially shaped as a torus,
preferably the outer end of the centrifuge container is a semi-torus.

**6.** The centrifuge container of claim 5, wherein the torus is a horn torus.

**7.** The centrifuge container of any of claims 5 to 6, wherein a centre of the torus is aligned with an outlet of the flushing tube,
preferably the outlet of the flushing tube is adjacent to the centre of the torus, preferably the outlet of the flushing tube is co-planar with the centre of the torus.

**8.** The centrifuge container of any of claims 5 to 7, wherein the torus has:

(a) a minor diameter of at least 0.5 times a diameter of the flushing tube, preferably at least 1 times, more preferably at least 1.5 times; and/or
(b) a major diameter of at least 1 times a diameter of the flushing tube, preferably at least 1.5 times, more preferably at least 2 times.

**9.** The centrifuge container of any preceding claim, wherein the first fluid port, the second fluid port and the flushing tube are spaced from one another in a second direction, transverse to the first direction,
preferably the flushing tube is between the first fluid port and the second fluid port in the second direction.

**10.** The centrifuge container of claim 9, wherein the first fluid port comprises a declined section extending towards an inner wall of the centrifuge container.

**11.** The centrifuge container of any preceding claim, wherein the flushing tube is aligned with the longitudinal axis of the centrifuge container.

**12.** The centrifuge container of any preceding claim, wherein an inner surface and/or an outer surface of the centrifuge container is rotationally symmetric about the longitudinal axis of the centrifuge container.

**13.** A centrifuge comprising:

a centrifuge housing;
a centrifuge rotor rotationally mounted in the centrifuge housing to rotate about a centrifuge axis; and
a centrifuge container according to any preceding claim attached to the centrifuge rotor.

**14.** The centrifuge of claim 13, further comprising a plurality of centrifuge containers according to any of claims 1 to 12 attached to the centrifuge rotor, each centrifuge container rotationally spaced about the centrifuge axis from one another.

**15.** A method of operating the centrifuge of claim 13 or 14, comprising the steps of:

flowing a liquid containing suspended particles into the centrifuge container;
rotating the rotor at a first rotational speed to concentrate the suspended particles at an outer end of the centrifuge container as a concentrated pellet;
rotating the rotor at a second rotational speed and removing the liquid from the centrifuge container; and
flushing the concentrated pellet from the centrifuge container.

Fig. 1

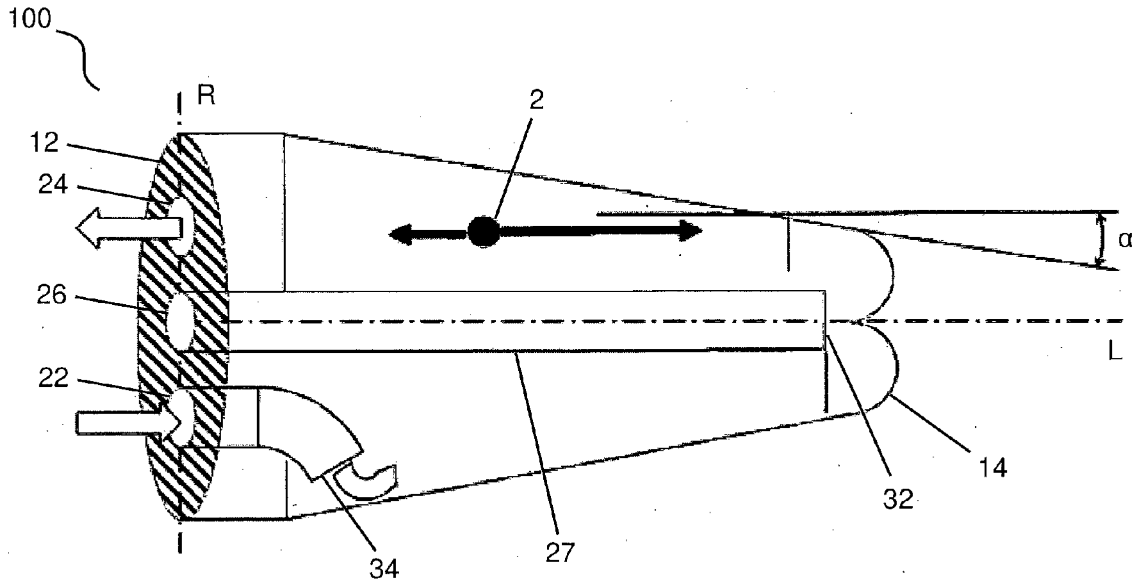


Fig. 2

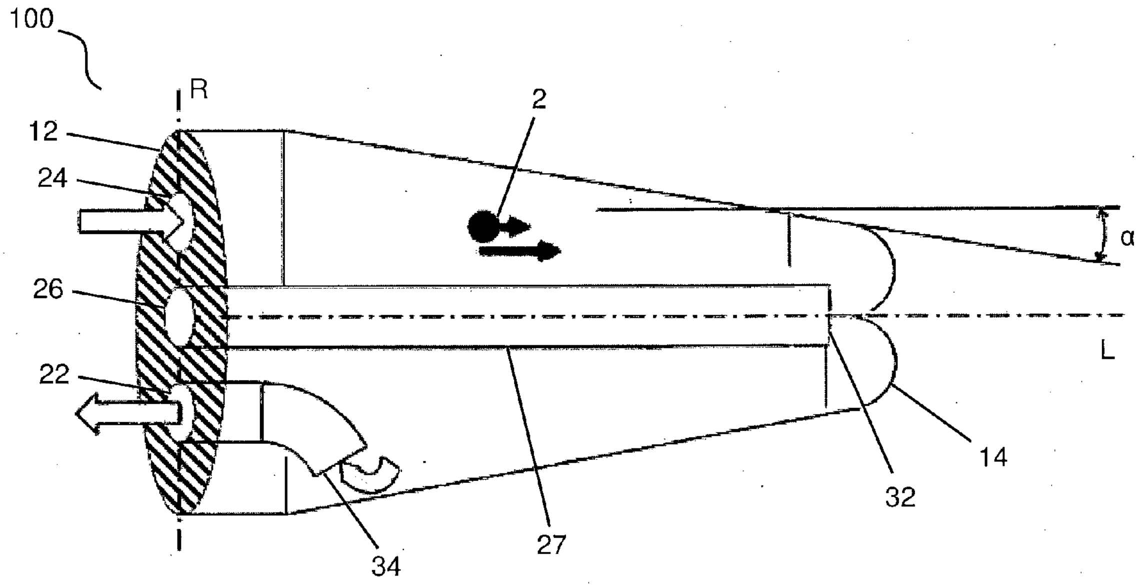

Fig. 3

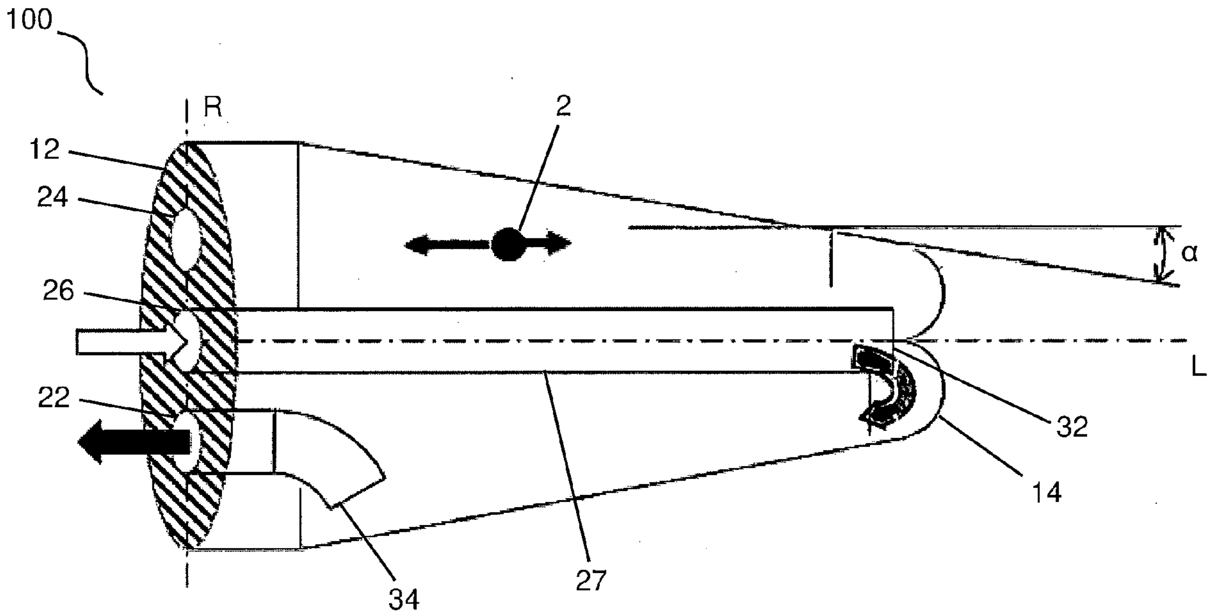

Fig. 4

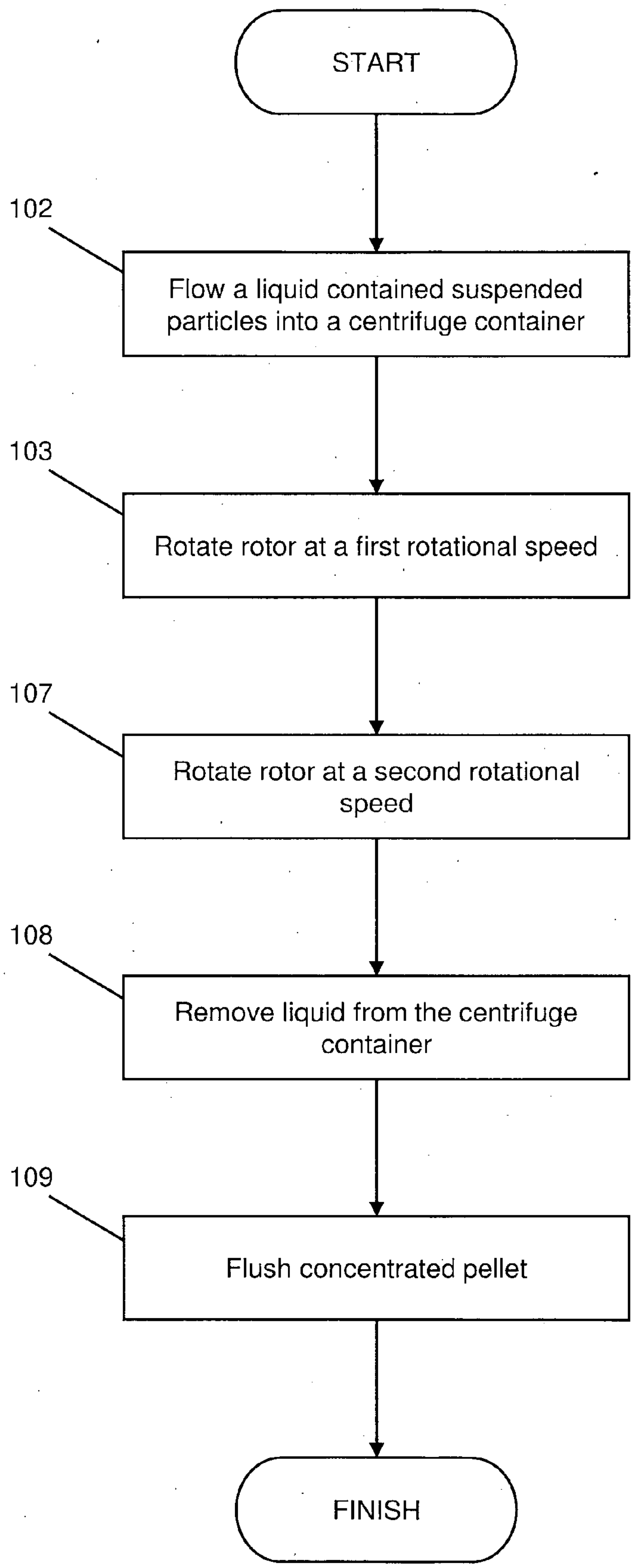
START
102
Flow a liquid contained suspended particles into a centrifuge container
103
Rotate rotor at a first rotational speed
107
Rotate rotor at a second rotational speed
108
Remove liquid from the centrifuge container
109
Flush concentrated pellet
FINISH

Fig. 5

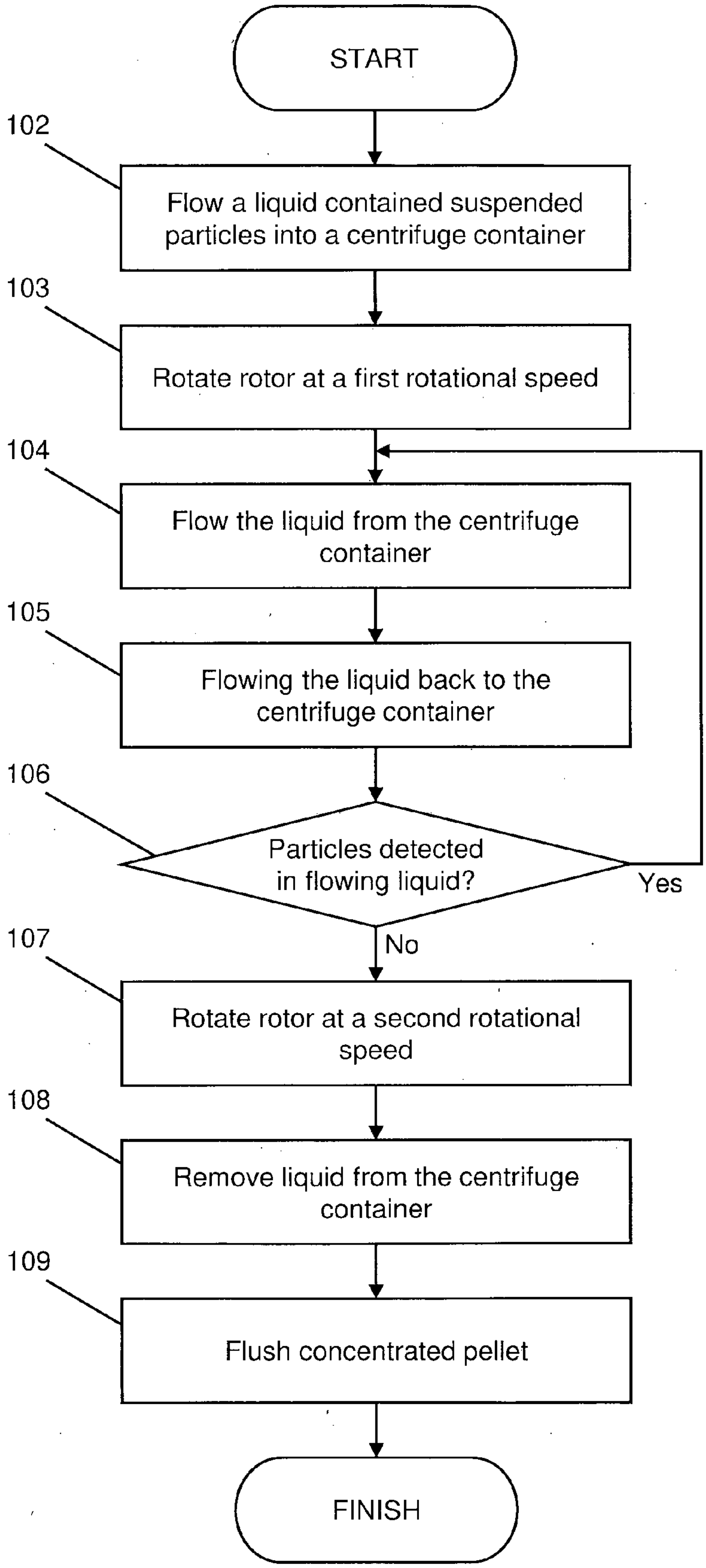
START
102
Flow a liquid contained suspended particles into a centrifuge container
103
Rotate rotor at a first rotational speed
104
Flow the liquid from the centrifuge container
105
Flowing the liquid back to the centrifuge container
106
Particles detected in flowing liquid?
Yes
No
107
Rotate rotor at a second rotational speed
108
Remove liquid from the centrifuge container
109
Flush concentrated pellet
FINISH

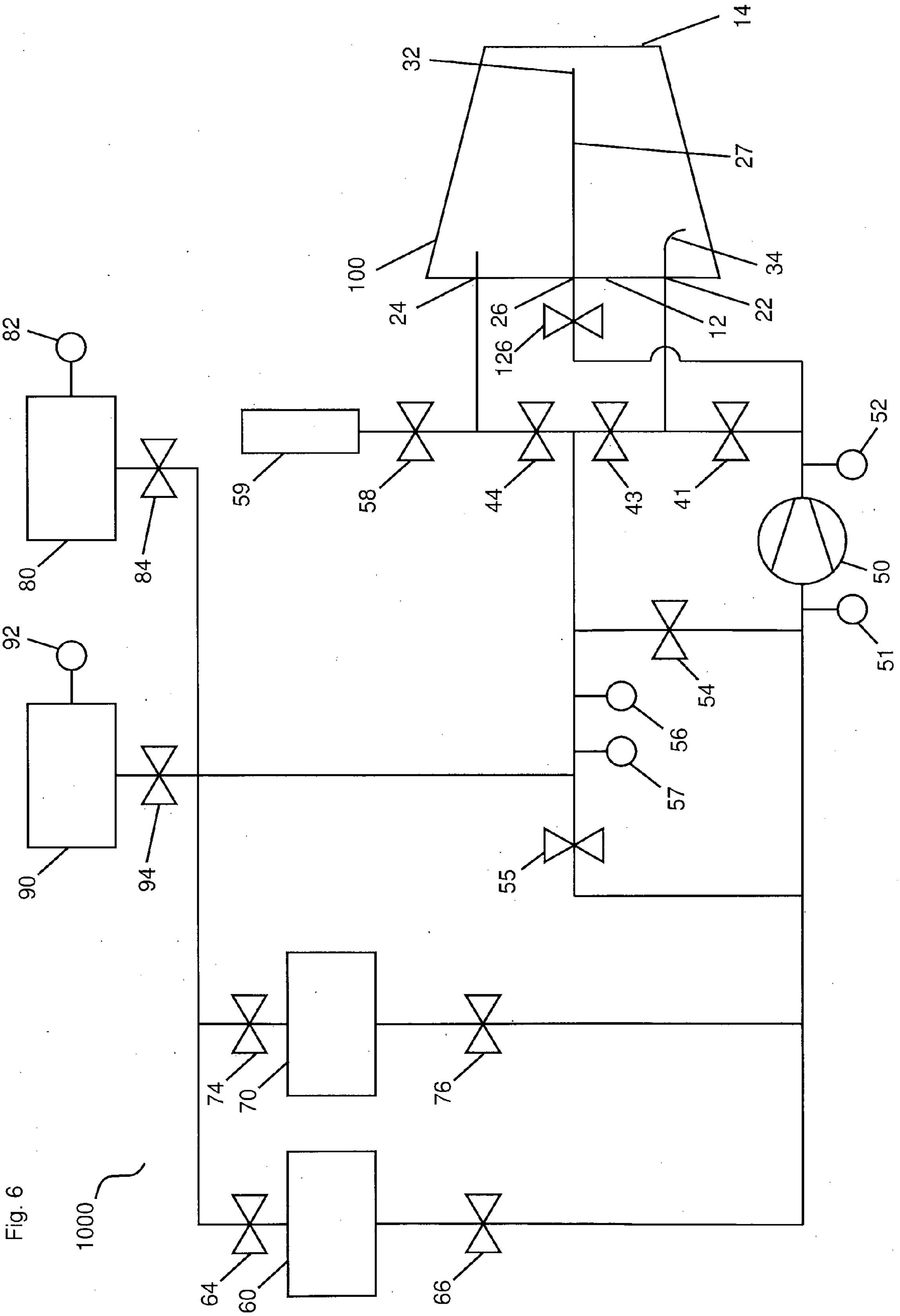
14
32
27
34
100
24
26
12
22
126
82
52
59
58
44
43
41
80
84
50
92
51
54
56
57
90
94
55
74
70
76
1000
64
60
66

Fig. 6

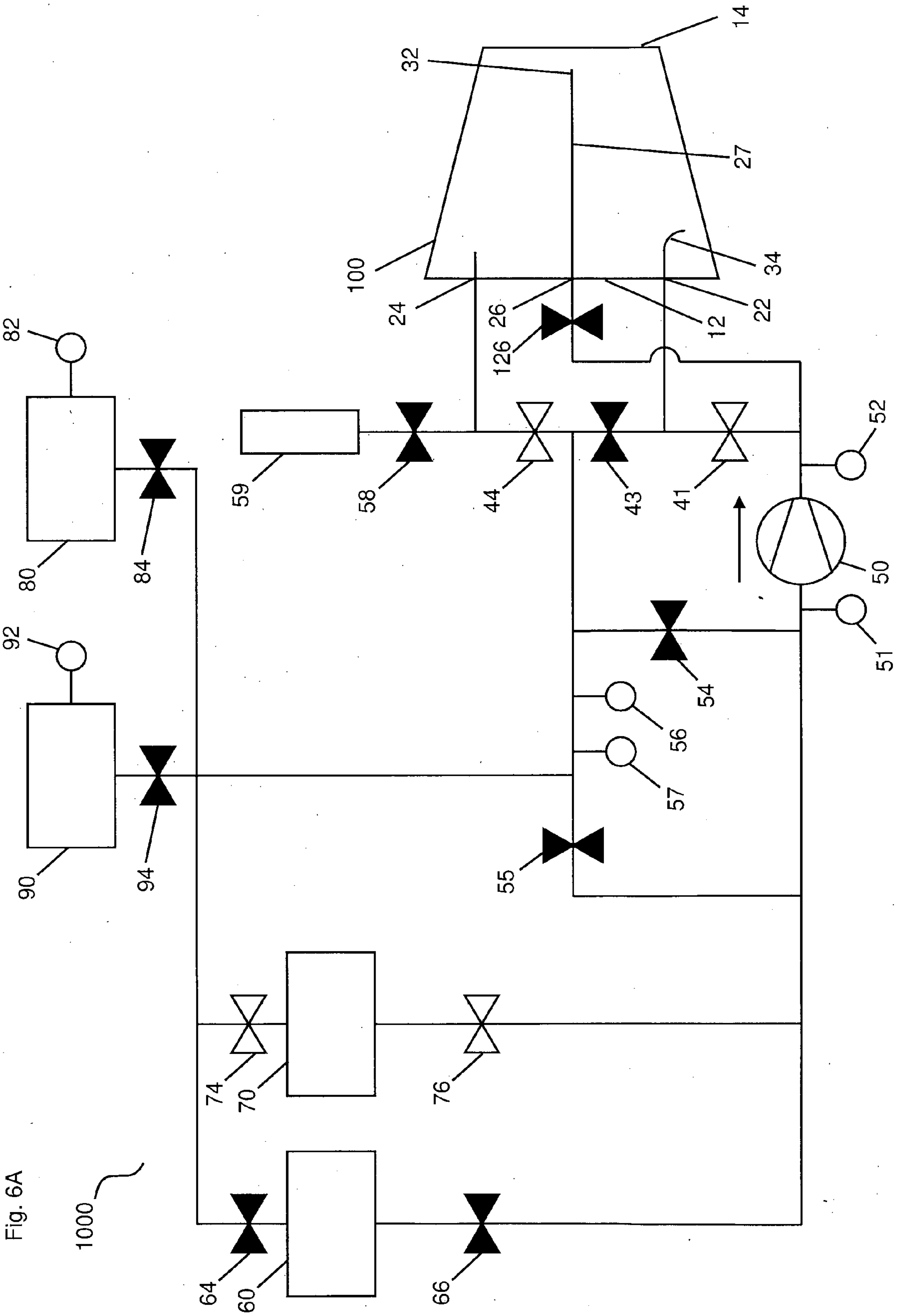
14
32
27
34
22
12
26
24
100
126
82
80
84
59
58
44
43
41
52
50
51
54
92
90
94
56
57
55
74
70
76
64
60
66
1000
Fig. 6A

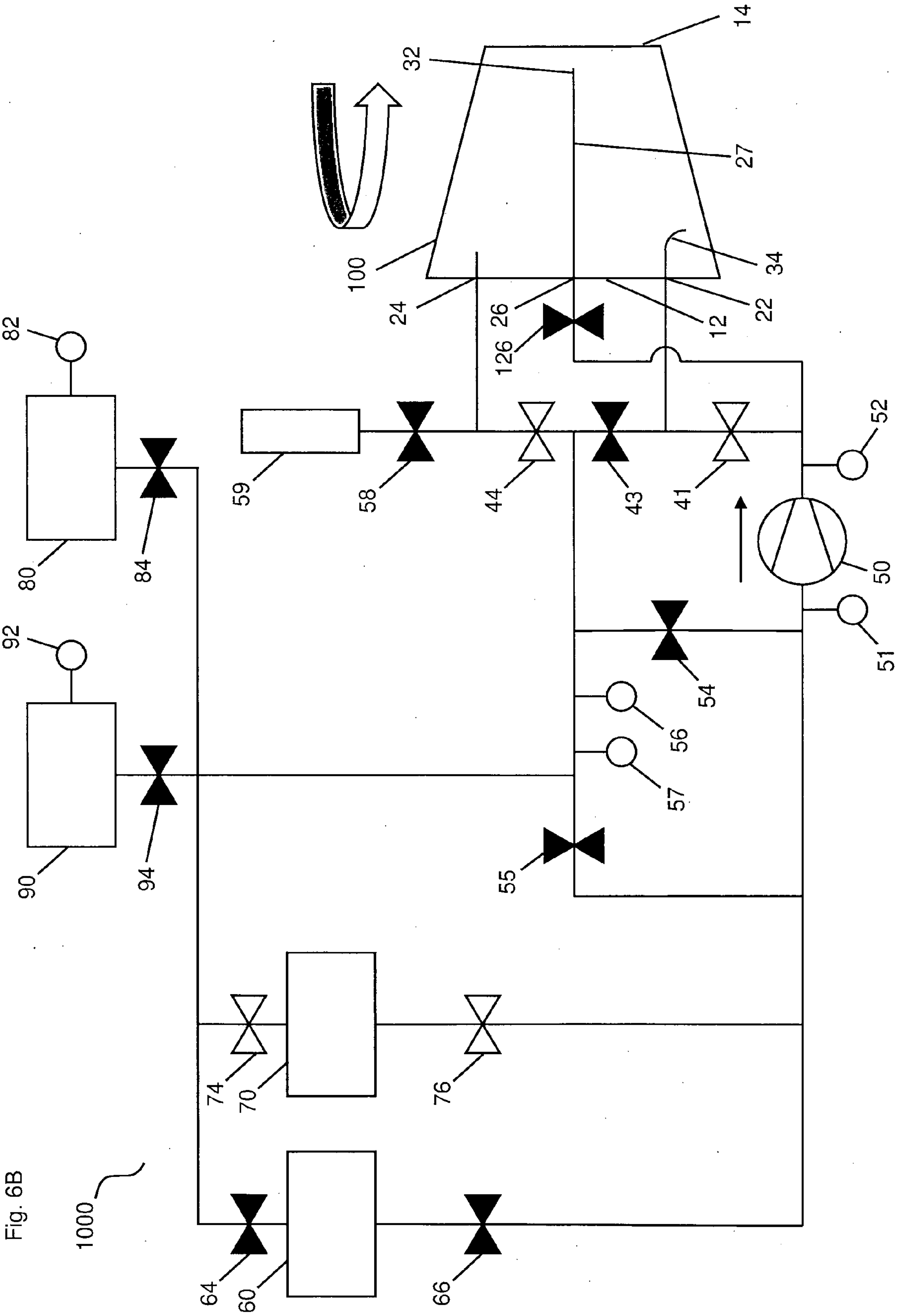
14
32
27
34
100
24
26
12
22
126
82
80
84
59
58
44
43
41
52
50
51
54
56
57
55
92
90
94
74
70
76
1000
64
60
66

Fig. 6B

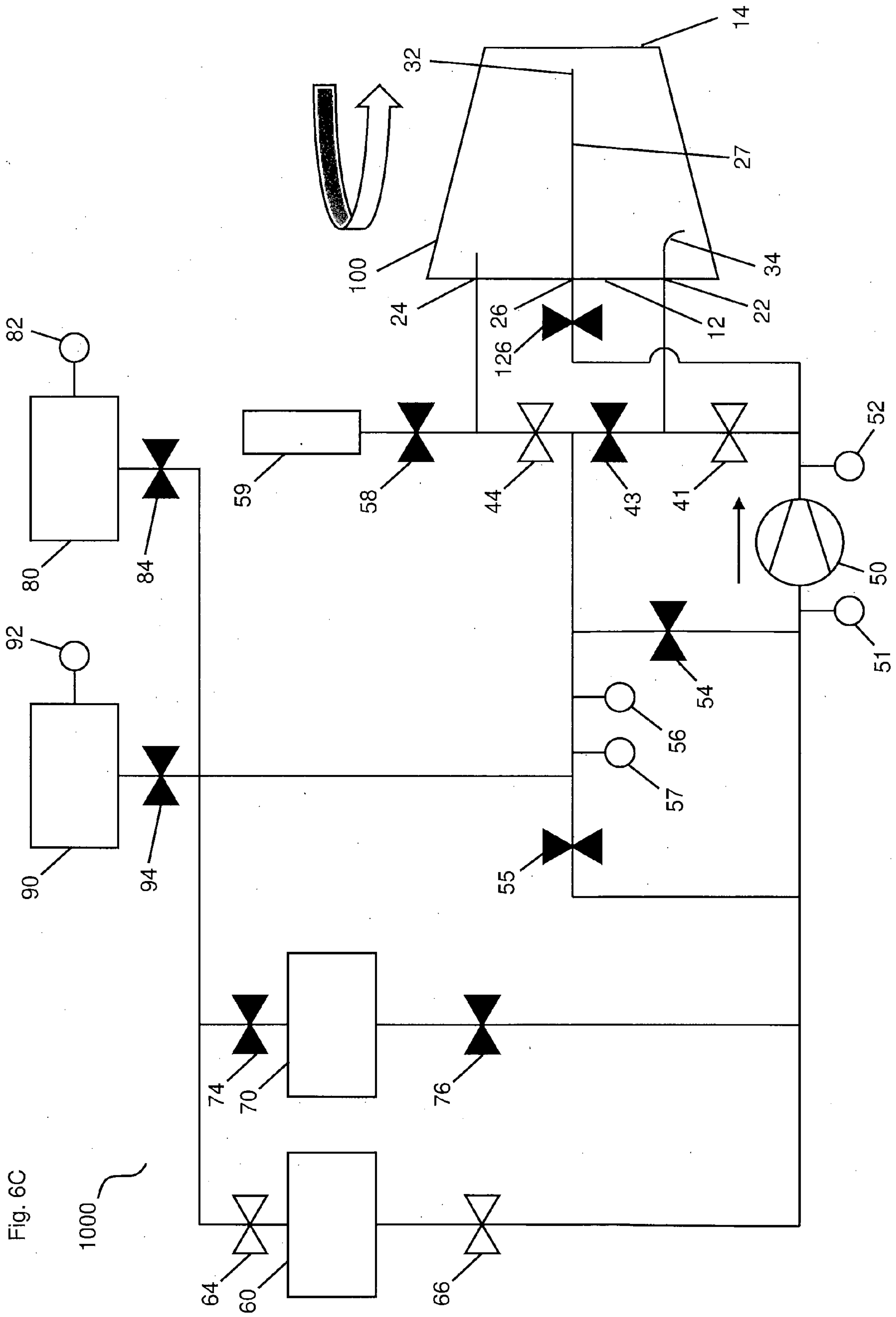
14
32
27
100
34
24
26
12
22
126
82
59
58
44
43
41
52
80
84
50
92
51
54
56
57
90
94
55
74
70
76
1000
64
60
66

Fig. 6C

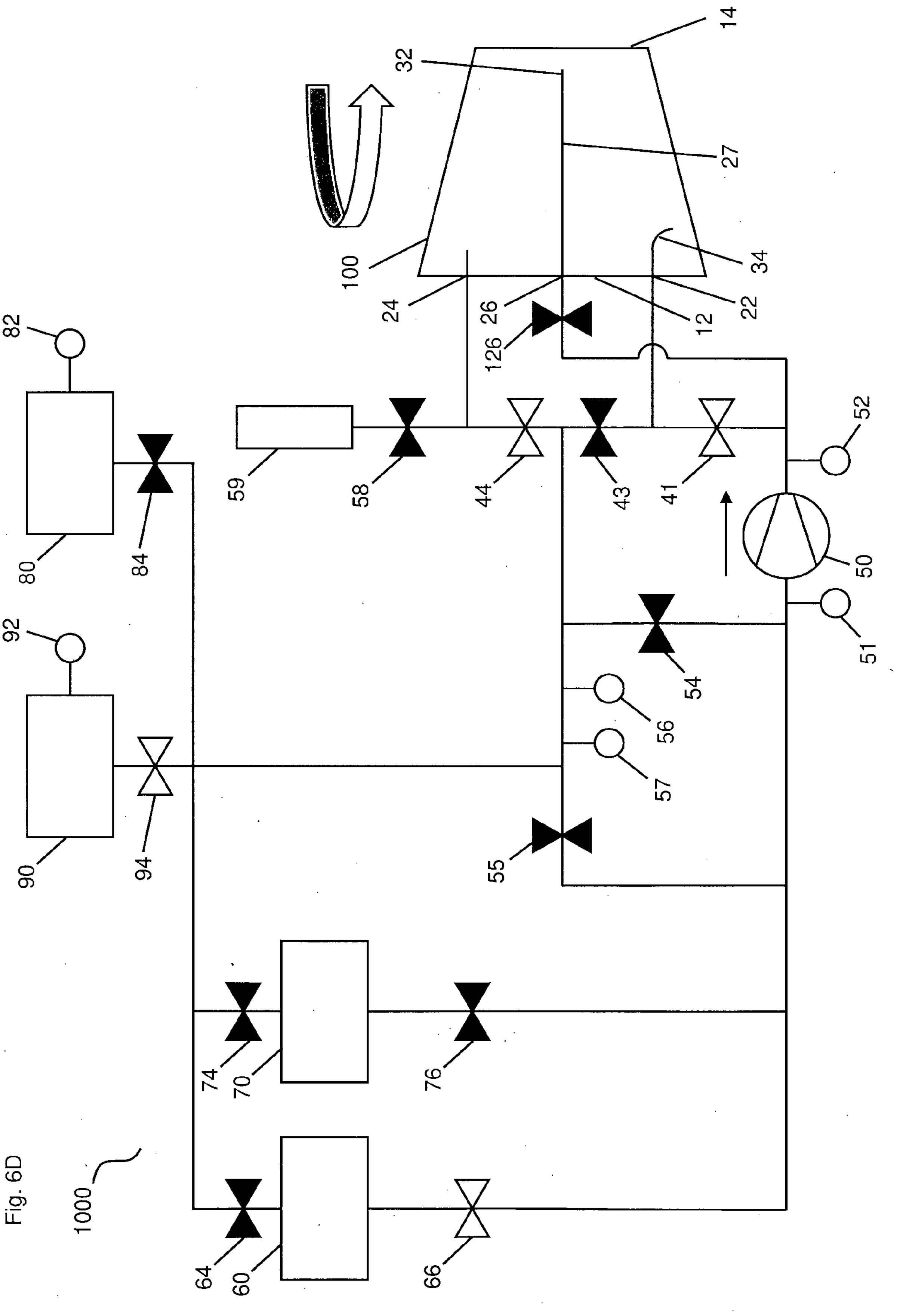
14
32
27
34
22
12
26
24
100
126
82
80
84
59
58
44
43
41
52
50
51
54
56
57
55
92
90
94
74
70
76
64
60
66
1000

Fig. 6D

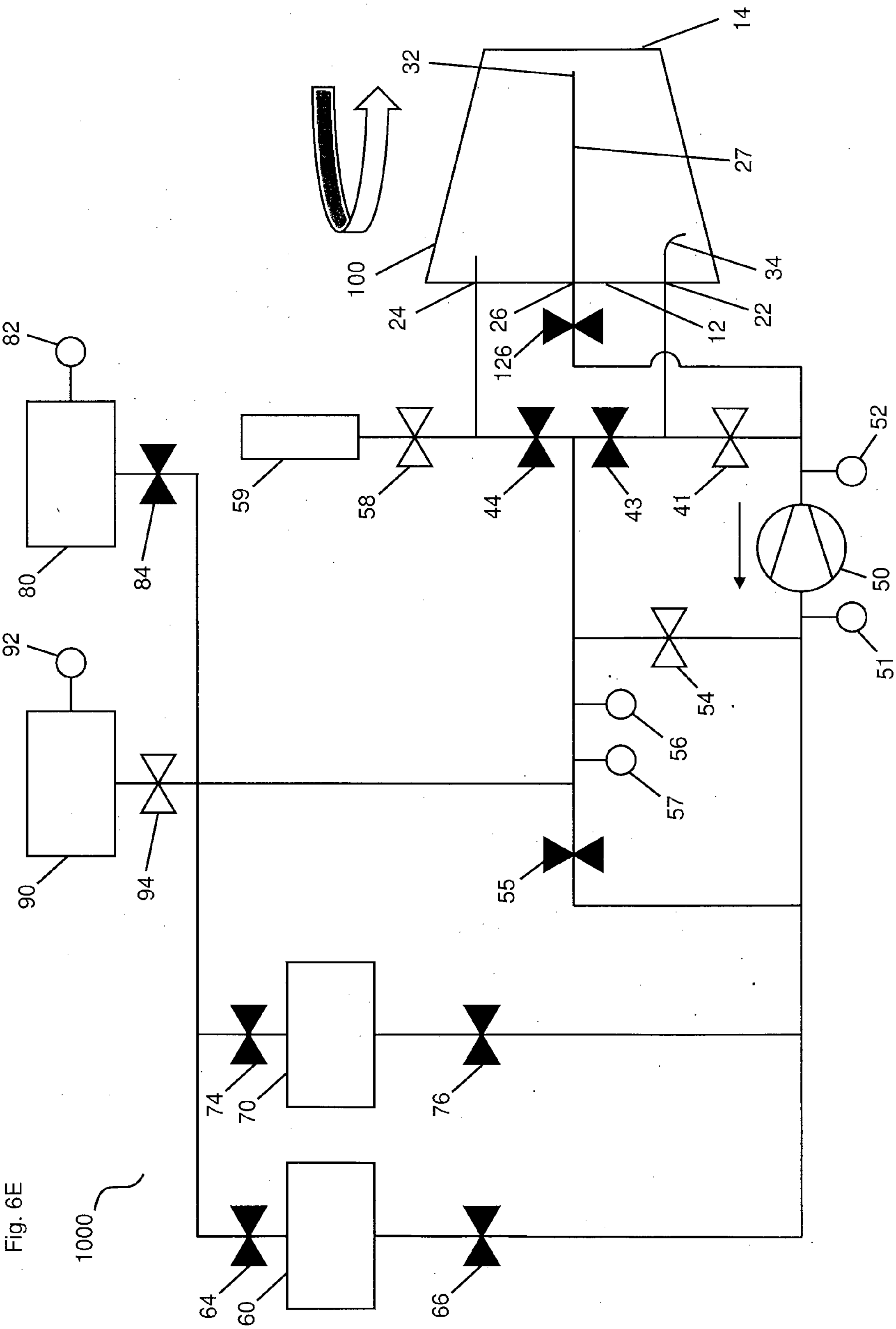

Fig. 6E

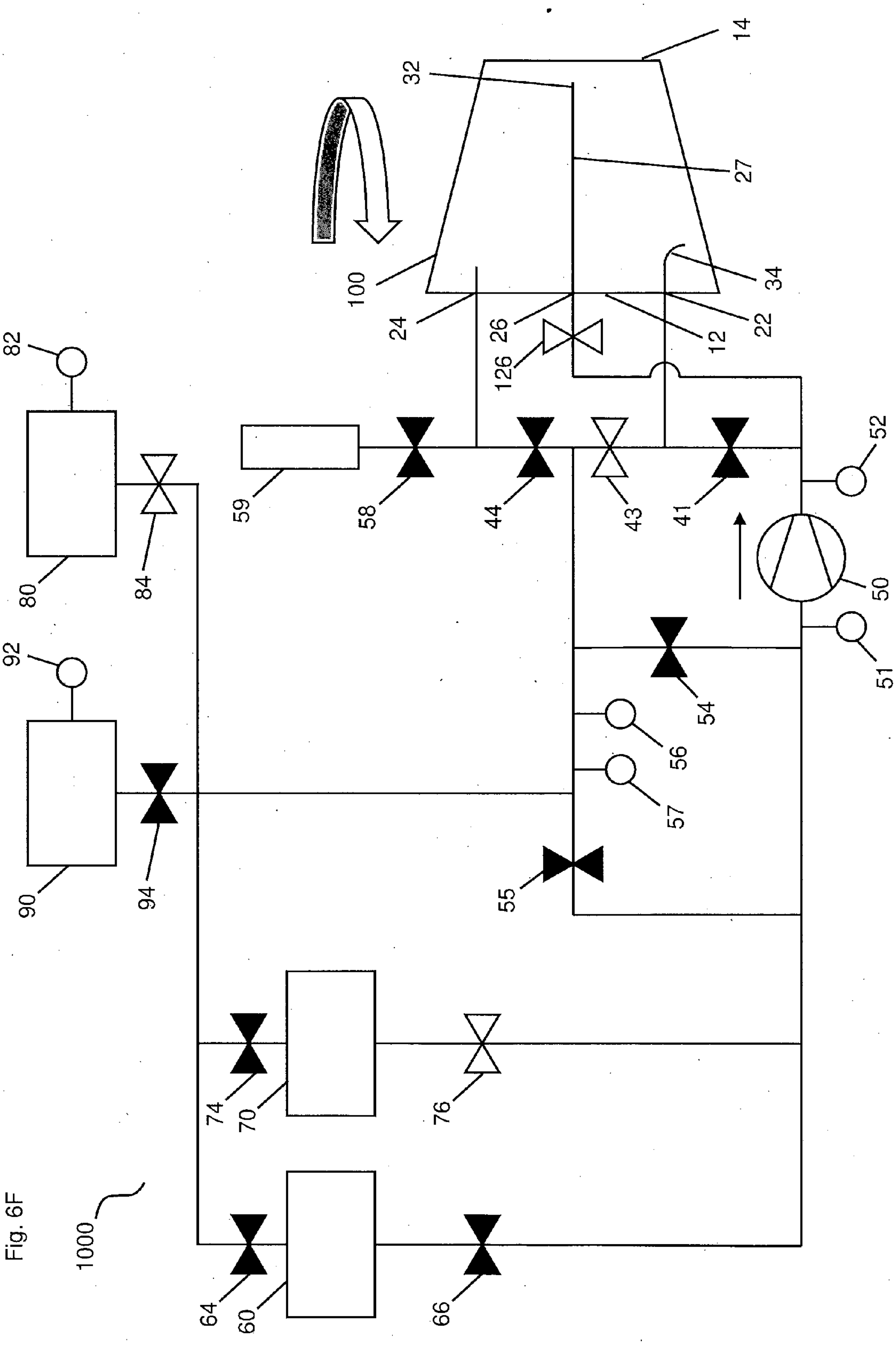
14
32
27
34
100
24
26
12
22
126
82
80
84
59
58
44
43
41
52
50
51
92
90
94
54
56
57
55
74
70
76
1000
64
60
66

Fig. 6F

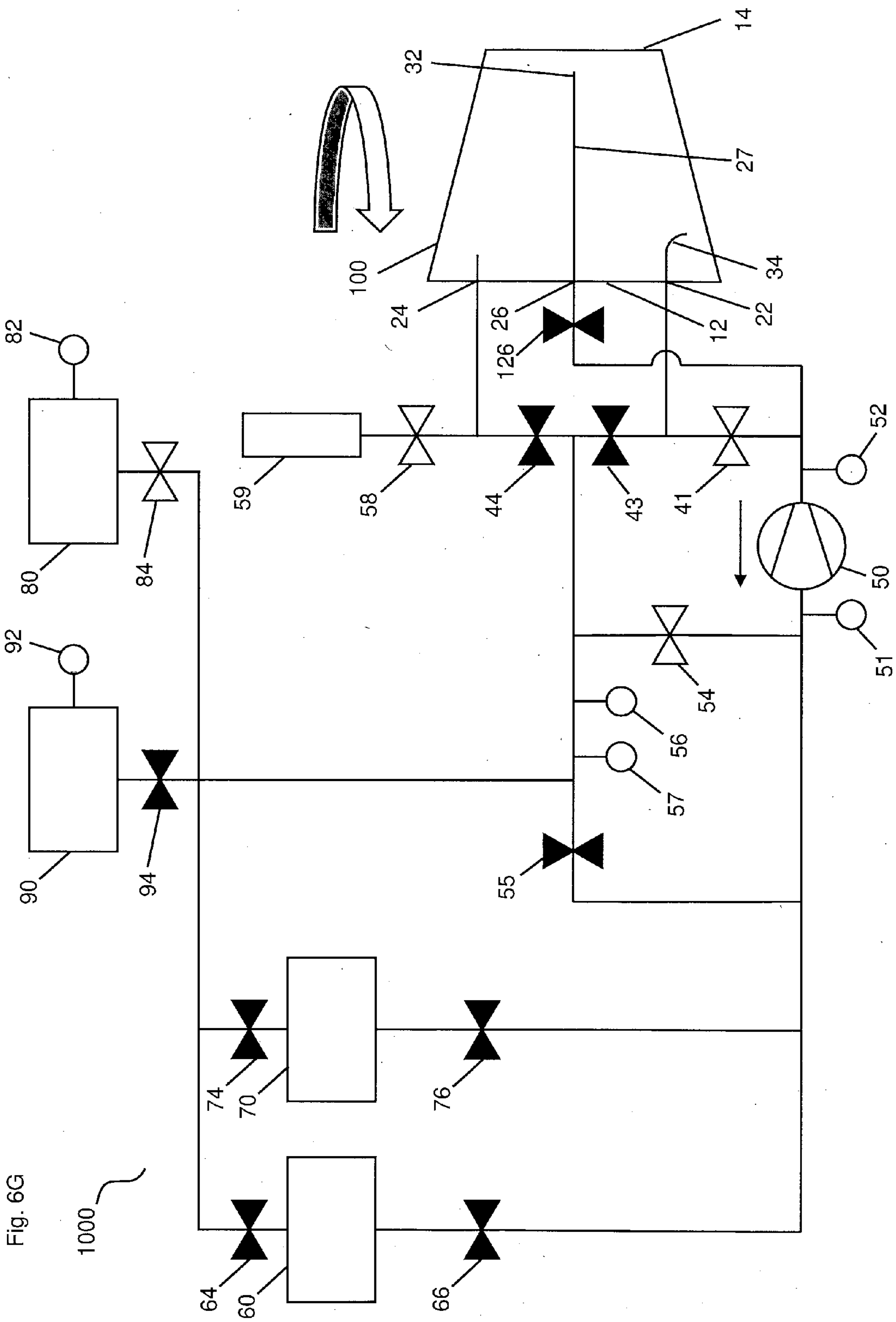
14
32
27
34
22
12
26
126
24
100
82
80
84
59
58
44
43
41
52
50
51
54
56
57
55
92
90
94
74
70
76
64
60
66
1000

Fig. 6G

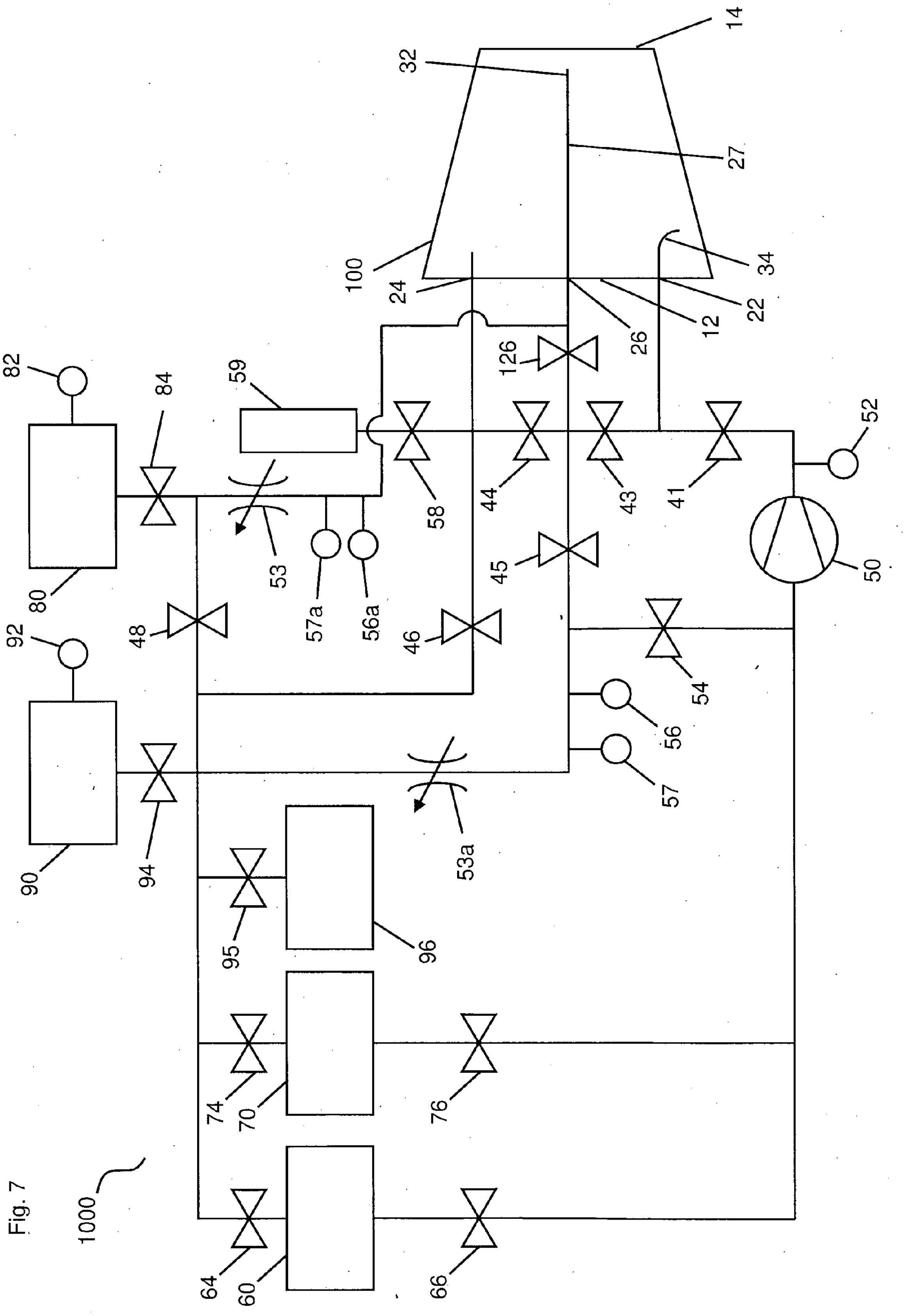
14
32
27
34
100
24
26
12
22
126
82
84
59
52
58
44
43
41
53
57a
56a
80
45
50
48
46
54
92
56
57
53a
90
94
95
96
74
70
76
1000
64
60
66

Fig. 7

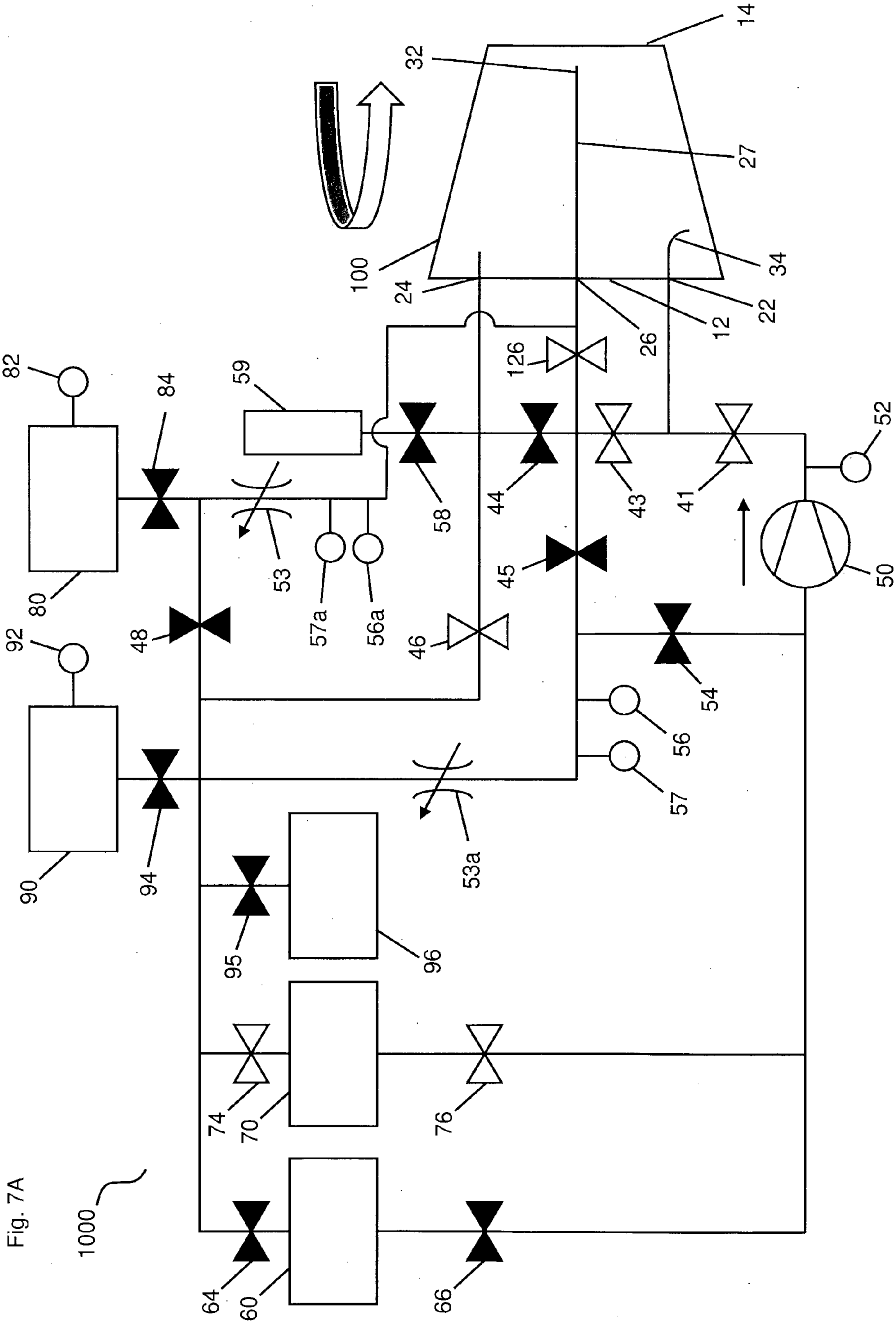
14
32
27
34
22
12
26
24
100
126
82
84
59
58
44
43
41
52
50
80
53
57a
56a
48
46
45
54
92
56
57
90
94
53a
95
96
74
70
76
1000
64
60
66

Fig. 7A

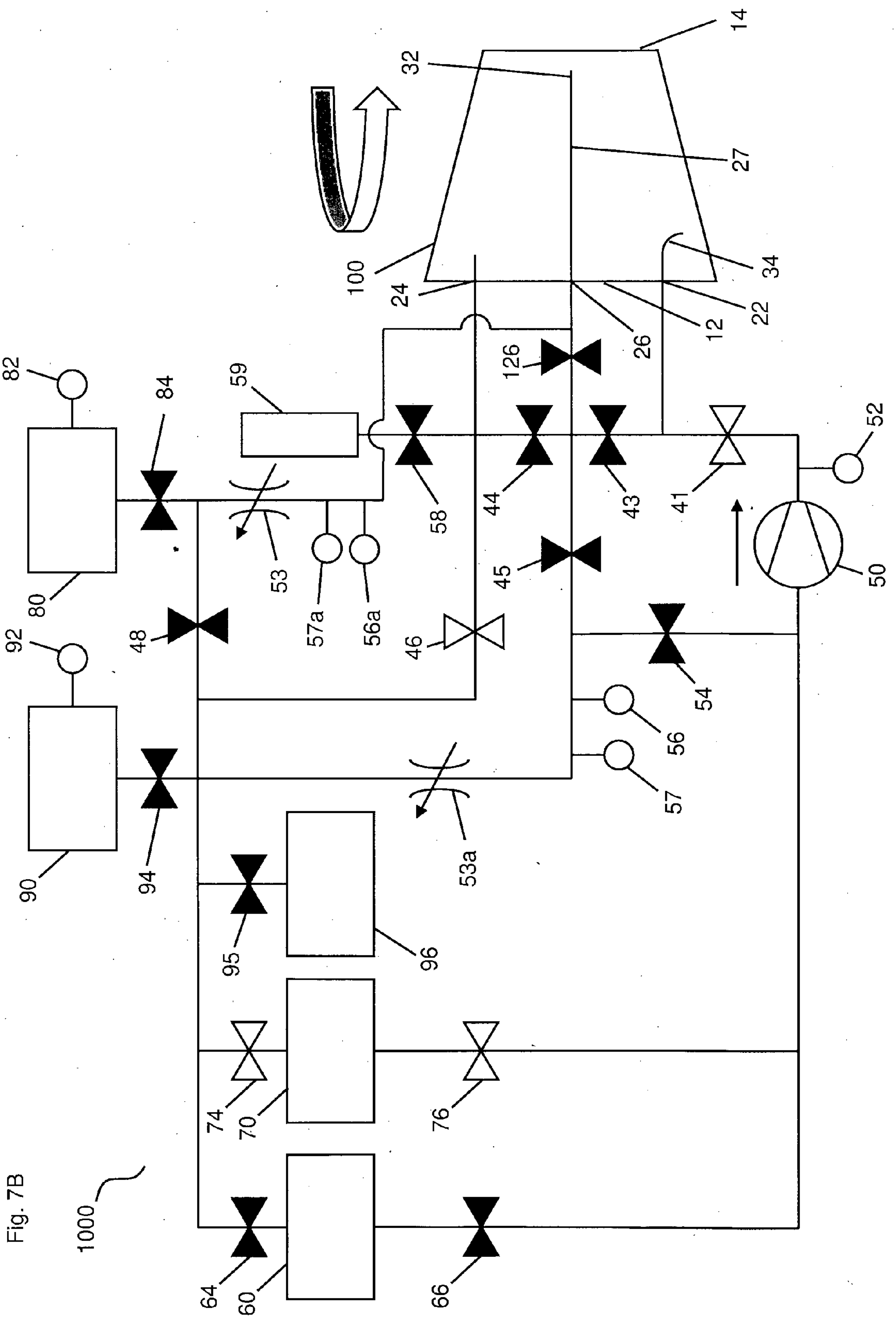
14
32
27
34
22
12
26
24
100
126
82
84
59
58
44
43
41
52
50
80
53
57a
56a
45
48
46
92
54
56
57
90
94
53a
95
96
74
70
76
1000
64
60
66

Fig. 7B

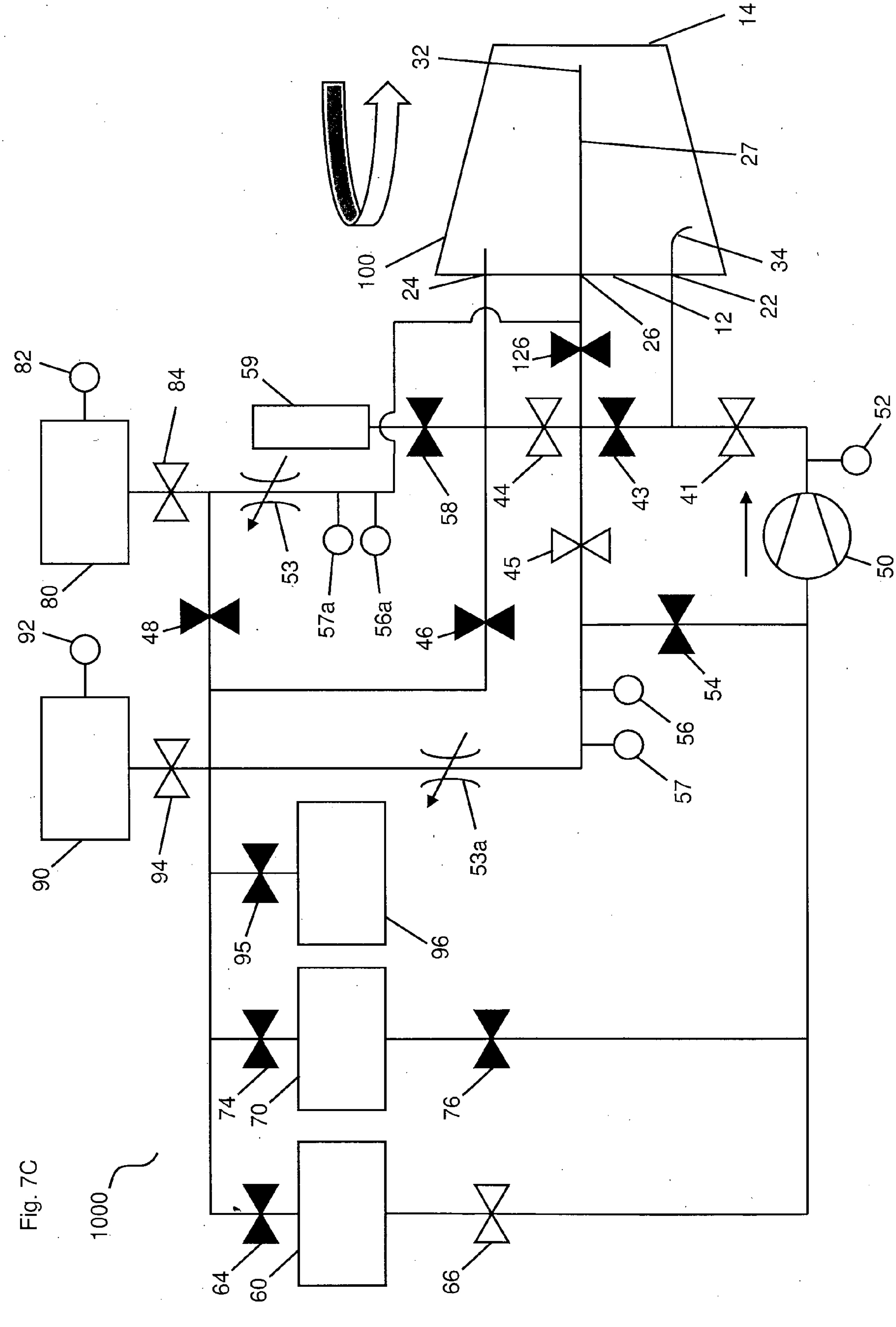
14
32
27
34
100
24
22
12
26
126
82
84
59
52
58
44
43
41
53
57a
56a
45
50
80
92
48
46
54
56
57
53a
90
94
95
96
74
70
76
1000
64
60
66

Fig. 7C

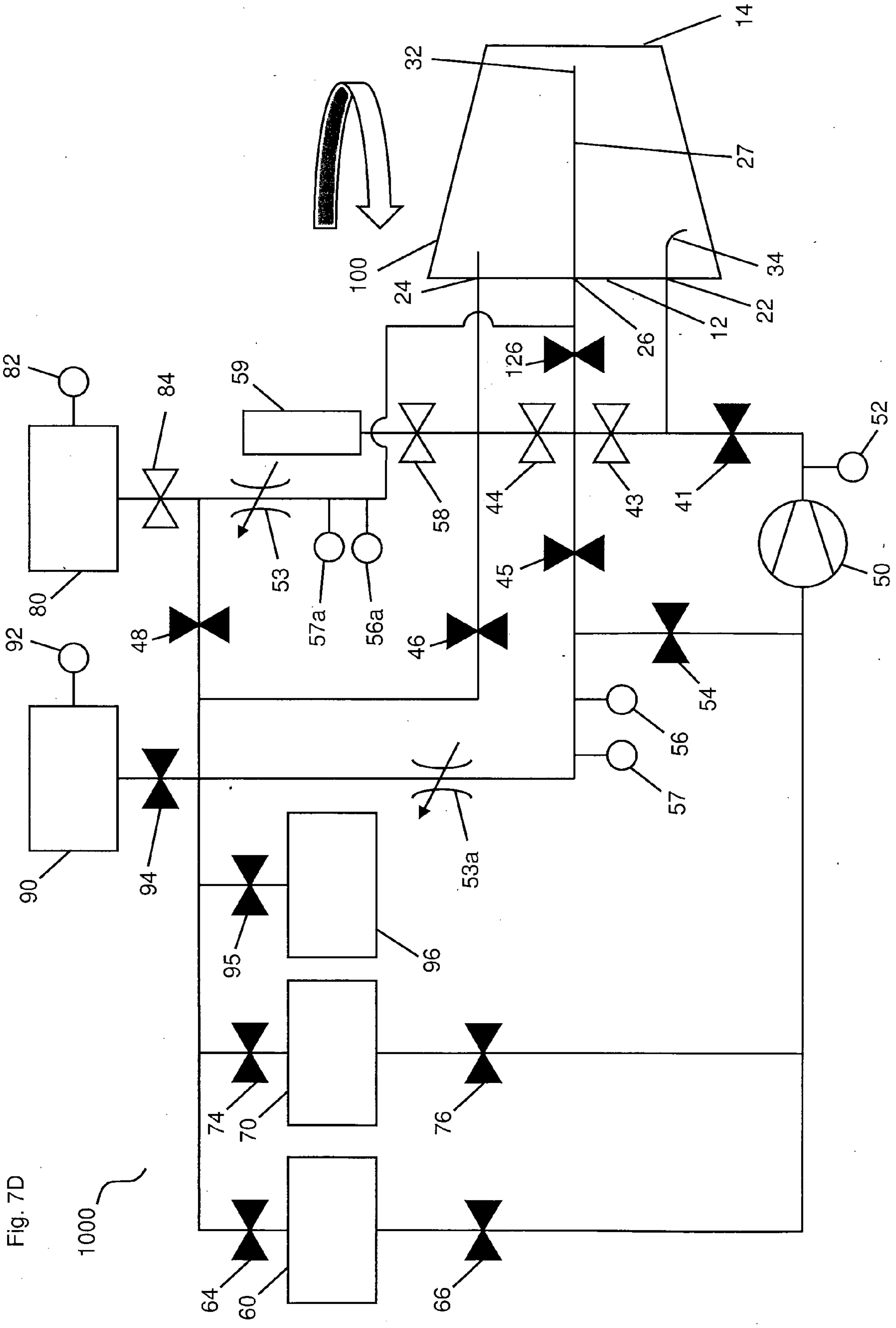
14
32
27
34
100
24
22
12
26
126
82
84
59
41
52
58
44
43
53
57a
56a
50
80
45
92
48
46
54
56
57
53a
90
94
95
96
74
70
76
1000
64
60
66

Fig. 7D

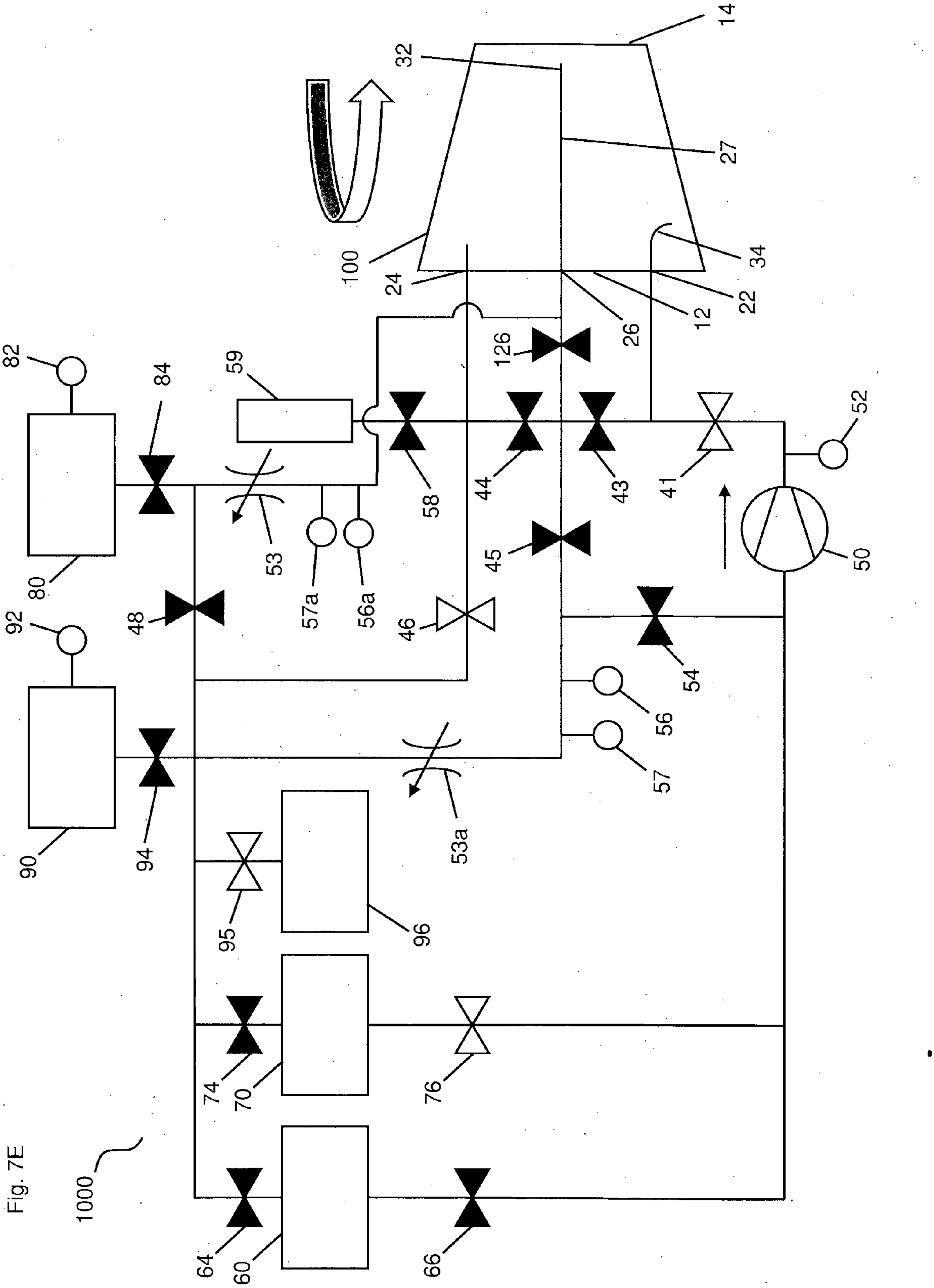
14
32
27
34
100
24
22
12
26
126
82
84
59
58
44
43
41
52
50
80
53
57a
56a
45
48
92
46
54
56
57
90
94
53a
95
96
74
70
76
1000
64
60
66

Fig. 7E

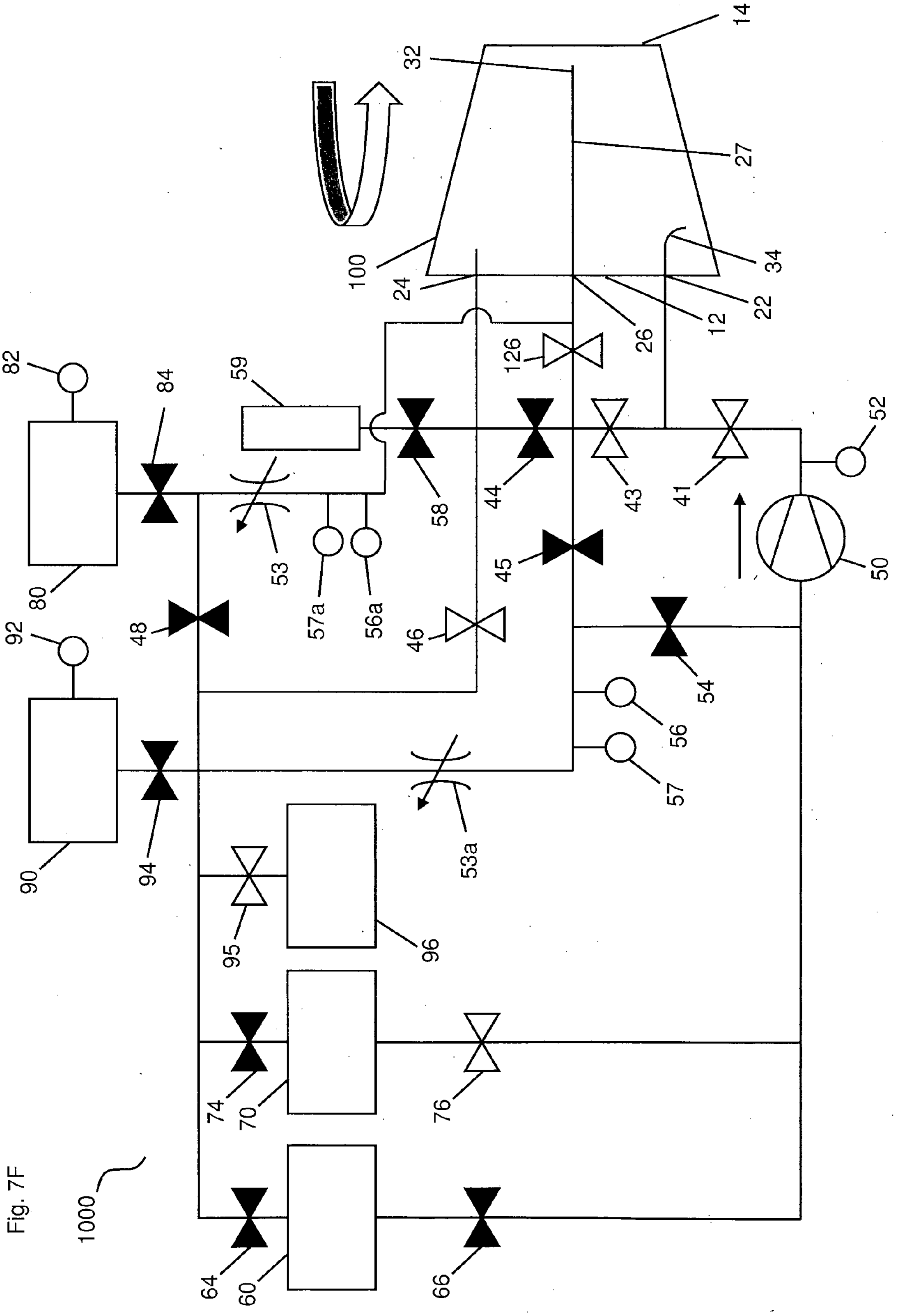

1000
14
32
27
34
100
24
26
12
22
126
82
84
59
58
44
43
41
52
50
80
53
57a
56a
48
45
46
54
92
56
57
90
94
53a
95
96
74
70
76
64
60
66


Fig. 7F

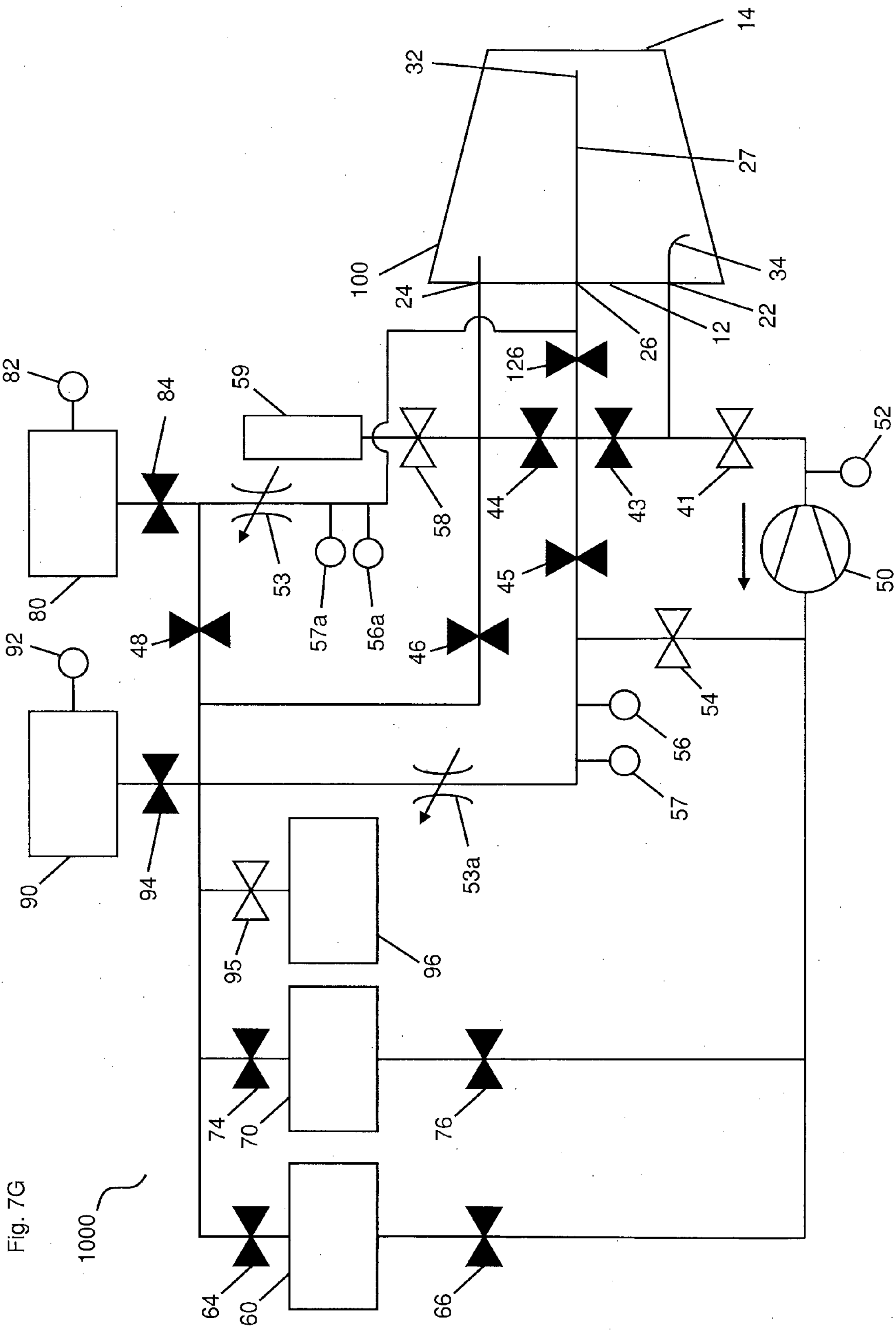
14
32
27
100
24
34
22
12
26
126
82
84
59
52
58
44
43
41
53
50
80
57a
56a
45
92
48
46
54
56
57
90
94
53a
95
96
74
70
76
1000
64
60
66

Fig. 7G

Europäisches Patentamt
European Patent Office
Office européen des brevets

# EUROPEAN SEARCH REPORT

Application Number
EP 23 31 5459

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP H04 247251 A (TOSHIBA CORP) 3 September 1992 (1992-09-03) | 1-5, 8-10,12, 13,15 | INV. B04B5/04 B04B5/02 B04B7/08 B04B11/02 B04B15/06 B01L3/00 C12M1/26 |
| A | * paragraphs [0002], [0079], [0085], [0093] - [0102]; figure 5 * | 6,7 | |
| X | WO 2005/030361 A1 (MITCHELSON KEITH RICHARD [AU]) 7 April 2005 (2005-04-07) | 1-4, 9-13,15 | |
| A | * p. 4, 5, bridging paragraph; figure 1 * | 6,7 | |
| X | EP 2 343 360 A1 (OLYMPUS CORP [JP]; CYTORI THERAPEUTICS INC [US]) 13 July 2011 (2011-07-13) | 1-4,9-15 | |
| A | * paragraph [0017]; figures 1-10 * | 6,7 | |
| A | WO 2021/191215 A1 (FRESH WORKS LTD [GB]) 30 September 2021 (2021-09-30) * page 2, lines 19-30; figure 8 * | 6,7 | |

TECHNICAL FIELDS SEARCHED (IPC)

B04B
B01D
B01L
C12M
A61M

The present search report has been drawn up for all claims

1

| Place of search | Date of completion of the search | Examiner |
|---|---|---|
| Munich | 18 April 2024 | Pössinger, Tobias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.........................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 23 31 5459

This annex lists the patent family members relating to the patent documents cited in the above-mentioned European search report.
The members are as contained in the European Patent Office EDP file on
The European Patent Office is in no way liable for these particulars which are merely given for the purpose of information.

18-04-2024

| Patent document cited in search report | Publication date | Patent family member(s) | Publication date |
|---|---|---|---|
| JP H04247251 A | 03-09-1992 | NONE | |
| WO 2005030361 A1 | 07-04-2005 | CN 1859961 A | 08-11-2006 |
| | | EP 1677889 A1 | 12-07-2006 |
| | | US 2007199907 A1 | 30-08-2007 |
| | | WO 2005030361 A1 | 07-04-2005 |
| EP 2343360 A1 | 13-07-2011 | EP 2343360 A1 | 13-07-2011 |
| | | JP 2010075066 A | 08-04-2010 |
| | | US 2011230328 A1 | 22-09-2011 |
| | | WO 2010035709 A1 | 01-04-2010 |
| WO 2021191215 A1 | 30-09-2021 | CA 3175902 A1 | 30-09-2021 |
| | | CN 115698416 A | 03-02-2023 |
| | | EP 4127302 A1 | 08-02-2023 |
| | | GB 2593465 A | 29-09-2021 |
| | | JP 2023518961 A | 09-05-2023 |
| | | KR 20230005171 A | 09-01-2023 |
| | | US 2023347267 A1 | 02-11-2023 |
| | | WO 2021191215 A1 | 30-09-2021 |

EPO FORM P0459

For more details about this annex : see Official Journal of the European Patent Office, No. 12/82